# EUROPEAN PATENT SPECIFICATION

(11) **EP 3 665 706 B1**
(45) Date of publication and mention of the grant of the patent: **02.08.2023**
(21) Application number: 18755676.6
(22) Date of filing: 03.08.2018
(51) Int. Cl.: G16H 50/50, G16H 20/10

(54) **GRAPHICAL USER INTERFACE FOR PERSONALIZED PREDICTION MODELING**
GRAFISCHE BENUTZEROBERFLÄCHE ZUR PERSONALISIERTEN VORHERSAGEMODELLIERUNG
INTERFACE UTILISATEUR GRAPHIQUE POUR UNE MODÉLISATION PRÉDICTIVE PERSONNALISÉE

(30) Priority: 08.08.2017 US 201715671310
(43) Date of publication of application: 17.06.2020
(73) Proprietor: Amgen Inc., Thousand Oaks, California 91320-1799 (US)
(72) Inventor: BALABAN, David, J., Santa Rosa Valley, CA 93012 (US); BROWN, Allen, L., Jr., Bellevue, WA 98004 (US); GNACADJA, Gilles, Hayward, California 94541 (US); TOUPIKOV, Mikhail, Agoura Hills, CA 91301 (US); DURST, Mark, J., San Leandro, CA 94577 (US); LEWIS-SANDY, Darrell, L., Bainbridge Island, WA 98100 (US)
(74) Representative: J A Kemp LLP
(86) International application number: PCT/US2018/045118
(87) International publication number: WO 2019/032391

(56) References cited:
- MARTÍNEZ-MARTÍNEZ JOSÉ M ET AL: "Prediction of the hemoglobin level in hemodialysis patients using machine learning techniques", COMPUTER METHODS AND PROGRAMS IN BIOMEDICINE, ELSEVIER, AMSTERDAM, NL, vol. 117, no. 2, 14 July 2014 (2014-07-14) , pages 208-217, XP029066840, ISSN: 0169-2607, DOI: 10.1016/J.CMPB.2014.07.001

## Description

### TECHNICAL FIELD

This application is generally related to developing a personalized prediction model, and, more specifically, to a system and method for adapting a general prediction model to an individual patient.

### BACKGROUND

Medical treatment of a patient is generally complicated and often times involves a strategic analysis of various risks and benefits. Changes made to label practices by regulatory drug administrations, e.g., U.S. Food and Drug Administration (FDA), may further complicate the treatment as well as affect medical personnel's, e.g., physicians, perceived complexity to treat the patient. As such, it may be difficult for a physician to conceptualize the risks and the benefits among various treatment options, as well as contextualize a risk-benefit profile of the each treatment for the patient.

Accordingly, computer models of physiological systems may be developed to assist physicians in determining treatment plans. These computer models may be utilized to predict how the physiological system will respond to various treatment events. Traditionally, these computer models seek to represent the physiological systems such that it is representative for an entire population. However, such techniques introduce a high variability into the underlying parameters that make up the physiological systems. Thus, there is a need to adapt a generalized model to accurately reflect how an individual patient will respond to the treatment events. MARTÍNEZ-MARTÍNEZ JOSÉ M ET AL, "Prediction of the hemoglobin level in hemodialysis patients using machine learning techniques", COMPUTER METHODS AND PROGRAMS IN BIOMEDICINE, ELSEVIER, AMSTERDAM, NL,Vol. 117, No. 2, 14 July, page 208-217 discloses a method of predicting hemoglobin levels in a patient.

### SUMMARY OF THE INVENTION

Example systems and methods for treating a patient are herein described, wherein a system and/or method for treating a patient with a medical condition related to an undesirable occurrence in at least one population of *in vivo* cells includes applying an adaptive dynamically predictive model to information associated with the patient, and treating the patient based on an output prediction from the model. In general, one or more aspects of the invention utilize model predictive control theory to model physiology, biology, and cell population dynamics to predict the impact of pharmaceuticals and other medical treatment on those populations. The models are adaptable to measurements of the underlying biological systems and thereby become personalized to individual patients. Formal specifications of software and/or hardware embodiments of the mathematical models may be constructed and logically verified to ensure accurate representation of a corresponding mathematical model. In some embodiments, mathematical models may be implemented as a simulator to enable a user to forecast a patient response, or as a controller to provide a user with mechanisms to construct strategies for achieving a desired patient response. Statistical verification may be utilized to verify that simulations of biological systems match experimental measurements made on such systems. Clinical protocols governing the therapeutic use of pharmaceuticals, including the interaction with the software systems modeling the biological systems to which the pharmaceuticals are being applied, may be formally specified, and protocols formalizing the therapeutic use of pharmaceuticals may be defined and logically verified to ensure that the defined protocol satisfies its specification. One or more of these aspects and/or criteria may be embodied in a smart device, e.g., code-generated provably-correct software and/or firmware.

More specifically, the system and/or method utilizes fully dynamic, adaptive, and/or predictive physiological mathematical models to predict one or more pivotal physiological properties of a patient given measurements of a limited number of physiological properties and any treatment option specified by a physician. This allows physicians to analytically evaluate multiple treatment options by examining the predicted effects of those options. Physicians can then use this predictive information to select the treatment that they believe is best. Additionally, since the mathematical model is adaptive, it adjusts its characterization of the patient each time a new measurement is made. In this way, the model very rapidly becomes personalized to the individual patient being treated. A few example applications are shown below in the following table:

| **Indication** | **Measured Physiological Property** | **Predicted Physiological Property** |
|---|---|---|
| Chemotherapy Induced Anemia (CIA) | Hemoglobin | Hemoglobin |
| Congestive heart Failure | Ejection Fraction, Heart Rate | Ejection Fraction |
| Chemotherapy Induced Thrombocytopenia | Platelet Count | Platelet Count |
| Hemodialysis Related Anemia | Hemoglobin, Iron | Hemoglobin |
| LDL Hyperlipidemia | LDL-C | LDL-C |
| Asthma | Exhaled Nitric Oxide | Inflammation Level Via Eosinophils |
| Chemotherapy Induced Neutropenia | WBC Count | WBC Count |
| Hyperparathyroidim | PTH, Vitamin D, Calcium, Phosphate | Calcium |

The invention is defined in the claims.

In accordance with a first example aspect of the invention directed to treating a patient with a medical condition related to an undesirable occurrence in at least one population of *in vivo* cells, the method comprises providing an adaptive dynamically predictive model for treating the patient; receiving, by one or more processors, a clinical value of a clinical event associated with the patient; receiving, by the one or more processors, a treatment strategy for treating the patient based on the clinical value; utilizing, by the one or more processors, the received treatment strategy and the adaptive dynamically predictive model and predicting a response of the patient to the treatment strategy; and displaying, by the one or more processors, a time profile of the predicted response of the patient to the treatment strategy.

In accordance with another example aspect of the invention directed to treating a patient with a medical condition related to an undesirable occurrence in at least one population of in vivo cells, a system comprises one or more processors communicatively coupled to an electronic medical record; a memory operatively coupled to the one or more processors; a user interface operatively coupled to the one or more processors; a treatment strategy module stored in the memory, which when executed on the one or more processors, provides a proposed treatment strategy at the user interface, including watchful waiting, watchful waiting with a transfusion, and dosing according to a label; and a prediction module stored in the memory, which when executed on the one or more processors, utilizes a selected treatment strategy to generate a result at the user interface including the patient's predicted response to the selected treatment strategy, wherein one or more generated results may be evaluated.

In accordance with further example aspect of the invention directed a tangible non-transitory computer-readable medium having instructions stored thereon for providing an adaptive dynamically predictive model for treating a patient with a medical condition related to an undesirable occurrence in at least one population of in vivo cells, the instructions, when executed by one or more processors of a computer system, cause the one or more processors to: receive a clinical value of a clinical event associated with the patient; receive a treatment strategy for treating the patient based on the clinical value; utilize the received treatment strategy and the adaptive dynamically predictive model and predict a response of the patient to the treatment strategy; and display a time profile of the predicted response of the patient to the treatment strategy.

### BRIEF DESCRIPTION OF THE DRAWINGS

Figure 1A is a diagram providing an overview of an example system and method for modeling, e.g., mathematical modeling, homeostasis in erythropoiesis (red blood cell (RBC) production).
Figure 1B is a flow diagram of an example method for adapting a generalized model to an individual patient.
Figure 1C is an example graphical representation of the a model producing a more accurate predication as it is adapted to a patient.
Figure 2 is a flow diagram of an example method for predicting a response to a proposed treatment for an example patient.
Figure 3 is an example chart displaying hemoglobin measurements of an example patient after two rounds of chemotherapy.

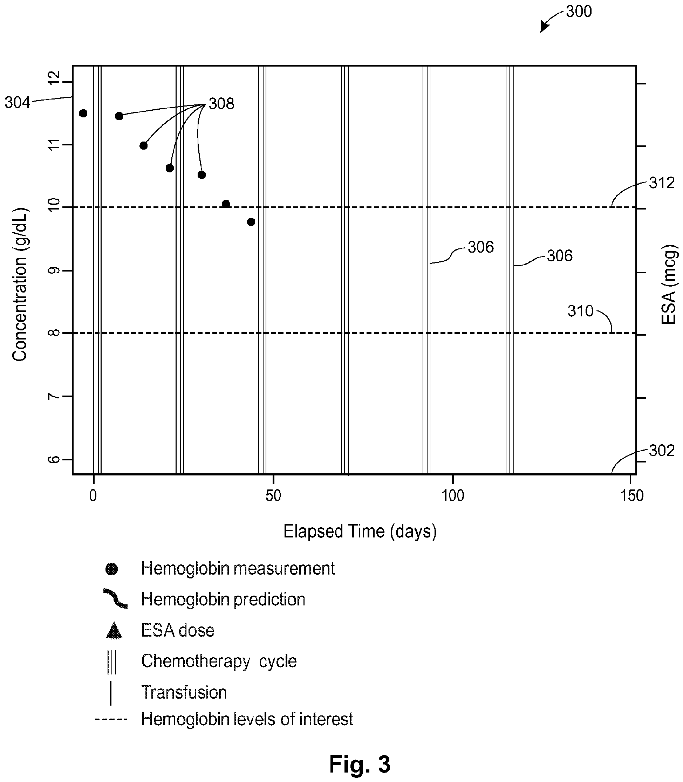

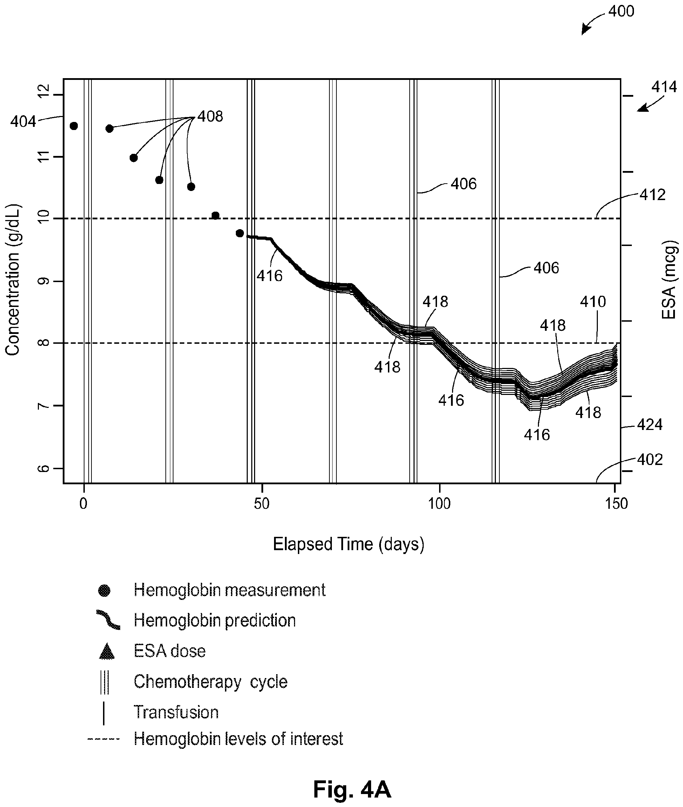

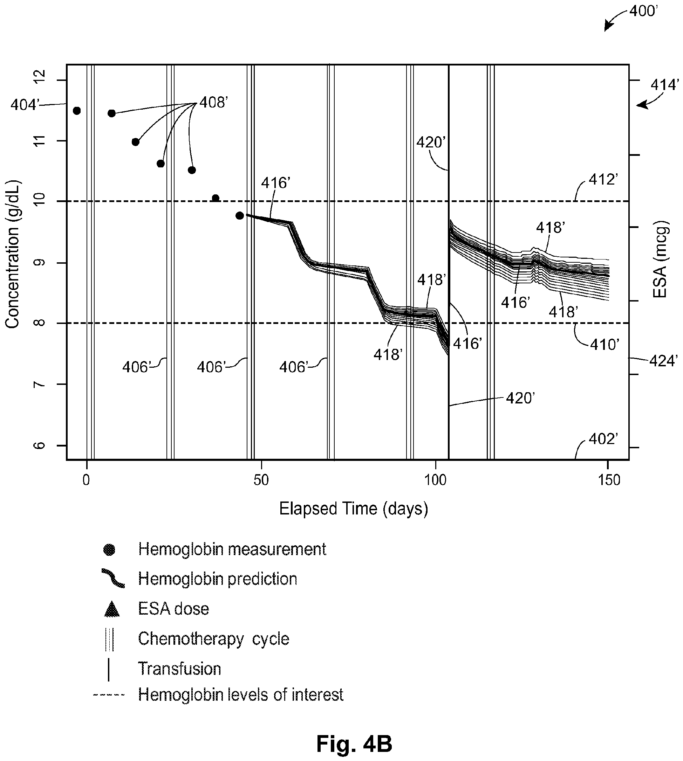

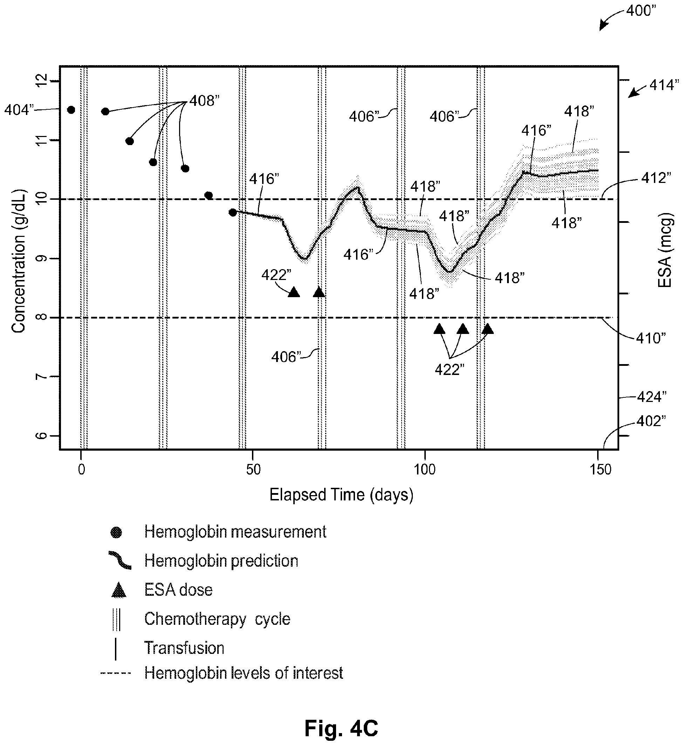

Figures 4A-4C are example charts displaying a predicted trend of hemoglobin concentration for the example patient associated with Figure 3 and being treated with a treatment strategy, wherein in Figure 4A the treatment strategy includes watchful waiting with consideration for later treatment after two rounds of chemotherapy; in Figure 4B the treatment strategy includes watchful waiting and a blood transfusion if the hemoglobin level is below 8 g/dL after two rounds of chemotherapy; and in Figure 4C the treatment strategy includes dosing an erythropoiesis-stimulating agent (ESA) according to the label after two rounds of chemotherapy.

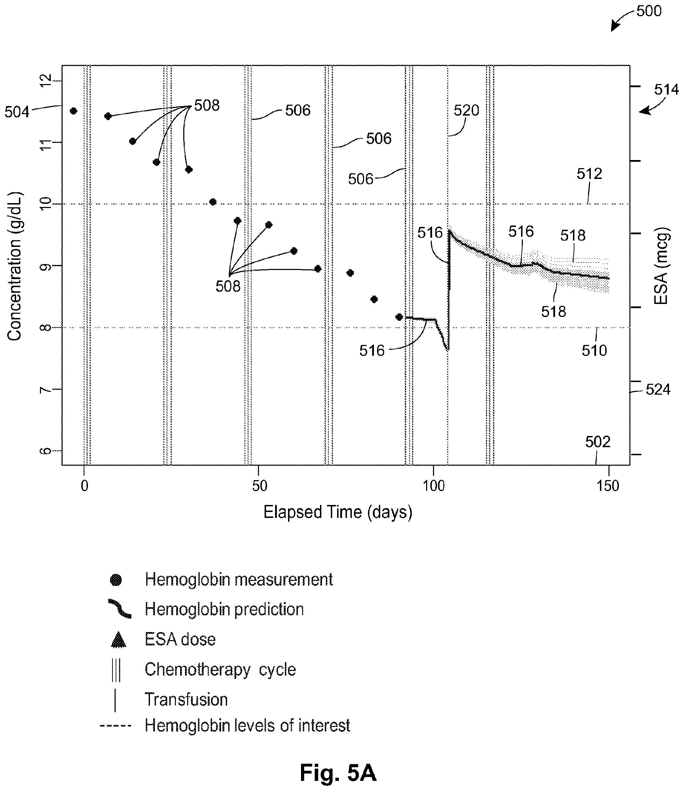

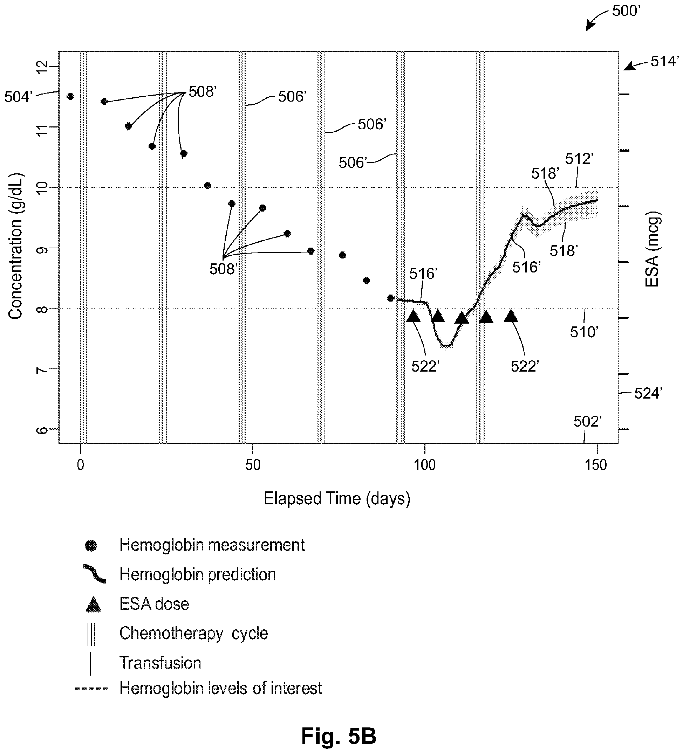

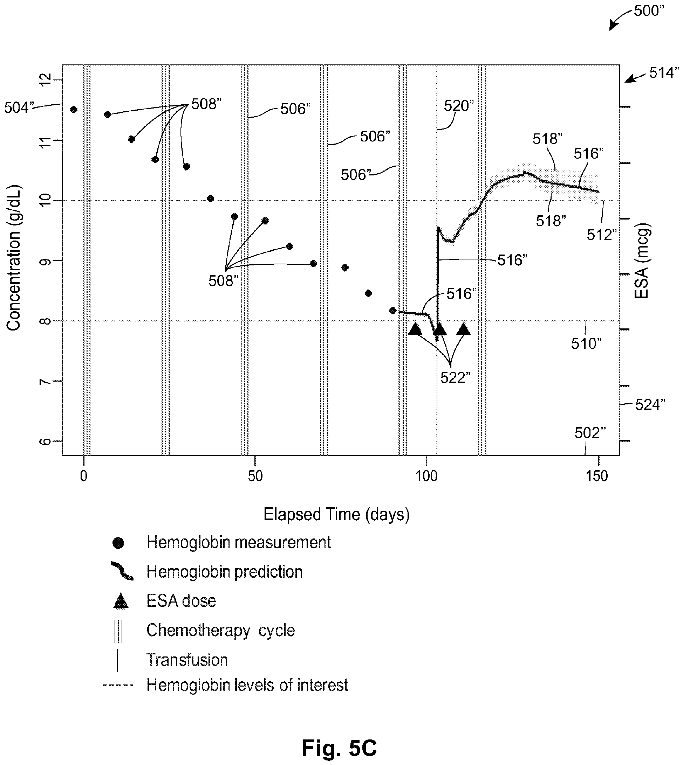

Figures 5A-5C are example charts displaying a predicted trend of hemoglobin concentration for the example patient associated with Figure 3 and being treated with a treatment strategy, wherein in Figure 5A the treatment strategy includes watchful waiting and a blood transfusion if the hemoglobin level is below 8 g/dL after two rounds of chemotherapy and again after four rounds of chemotherapy; in Figure 5B the treatment strategy includes watchful waiting with consideration for later treatment after two rounds of chemotherapy and dosing ESA according to the label after four rounds of chemotherapy; and in Figure 5C the treatment strategy includes watchful waiting and a blood transfusion if the hemoglobin level is below 8 g/dL after two rounds of chemotherapy and dosing ESA according to the label after four rounds of chemotherapy.
Figures 6A and 6B are example charts displaying a predicted trend of hemoglobin concentration for an example patient being treated with a treatment strategy, wherein in Figure 6A the treatment strategy includes watchful waiting with consideration for later treatment after two rounds of chemotherapy; and in Figure 6B the treatment strategy includes watchful waiting with consideration for later treatment after two rounds of chemotherapy and dosing ESA according to the label after four rounds of chemotherapy.

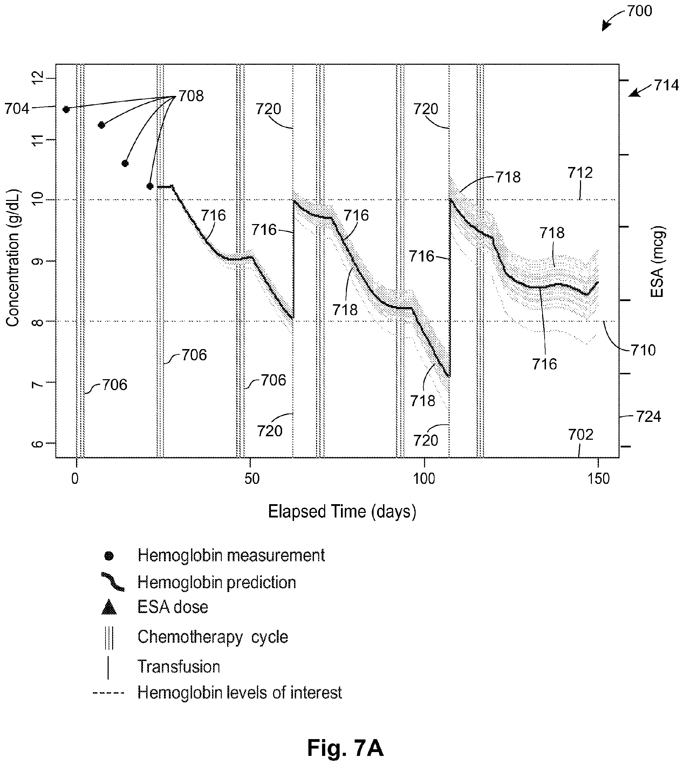

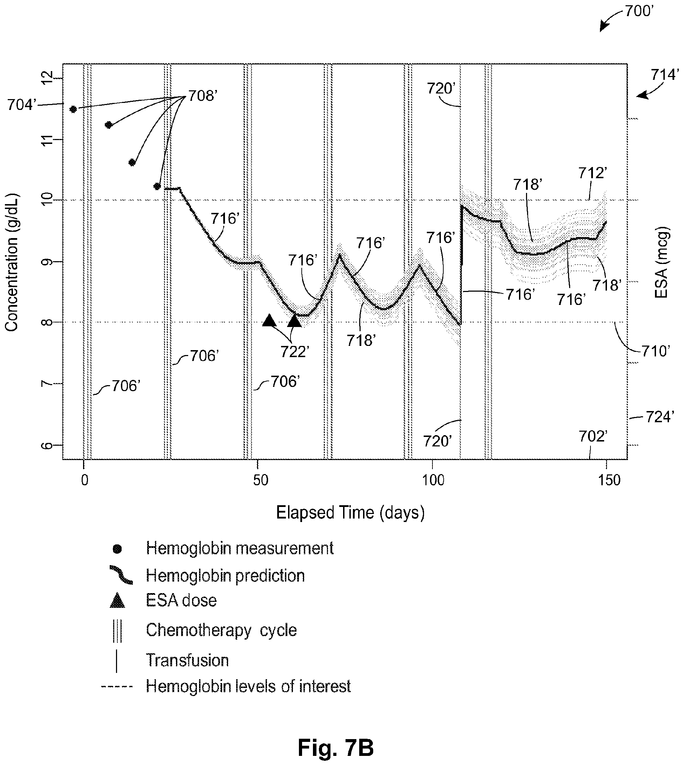

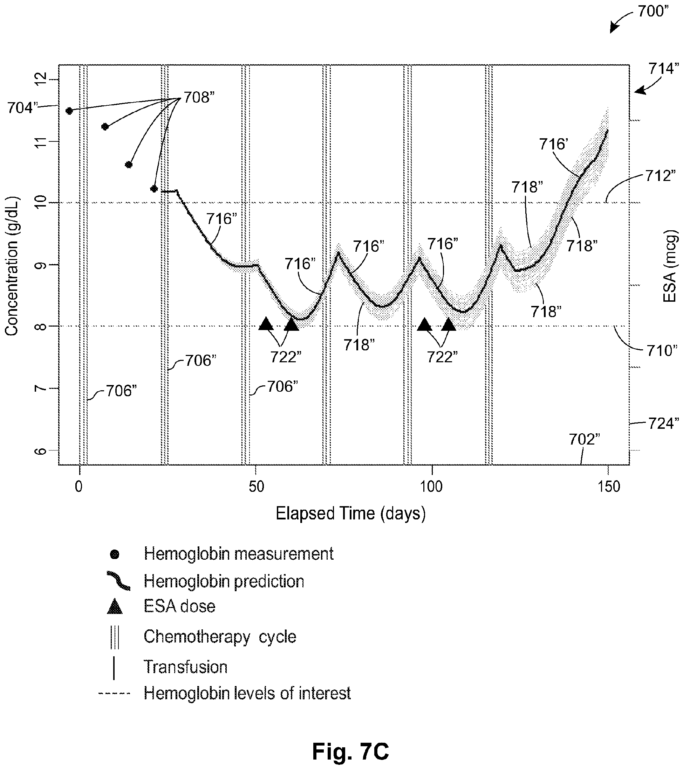

Figures 7A-7C are example charts displaying a predicted trend of hemoglobin concentration for an example patient being treated with a treatment strategy, wherein in Figure 7A the treatment strategy includes watchful waiting and consideration for later treatment after two rounds of chemotherapy; in Figure 7B the treatment strategy includes dosing ESA according to the label after two rounds of chemotherapy and then watchful waiting with consideration for later treatment; and in Figure 7C the treatment strategy includes dosing ESA according to the label after two rounds of chemotherapy and then to continue dosing ESA according to the label.
Figure 8 illustrates a block diagram of an example network and computer hardware that may be utilized with a system and/or method in accordance with the described embodiments.
Figure 9 illustrates a block diagram of an example computer system on which a system and method may operate in accordance with the described embodiments.

### DETAILED DESCRIPTION

The disclosed system and method utilize a personalized mathematical model based on physiological data to forecast an individual patient's response to a proposed medical treatment. The system and method is capable of being applied across a variety of indications and drugs. Some indications include, and are not limited to, anemia, including but not limited to chemotherapy induced anemia or radiation induced anemia, congestive heart failure, cytopenia, including but not limited to chemotherapy induced thrombocytopenia or radiation induced thrombocytopenia, hemodialysis related anemia, LDL hyperlipidemia, asthma, neutropenia, including chemotherapy induced neutropenia or radiation induced neutropenia, and hyperparathyroidism. The system and/or method predicts a trend of one or more physiological properties of the patient in response to one or more treatment strategies. If available, the system and method may utilize one or more measured physiological properties of the patient to predict the trend of the one or more physiological properties.

In one example embodiment, the system and/or method is used with patients susceptible to developing chemotherapy induced anemia (CIA). More specifically, the system and/or method predicts a trend of the patient's hemoglobin (Hg) concentration level in response to one or more proposed treatment strategies. In the early stage of treatment when patient information may be limited, the model may predict the individual patient's behavior response in alignment with that particular patient's cohort population. That is, when there is limited data available for an individual patient, the model can utilize known data from a stratified population associated with the individual patient to predict the patient's hemoglobin trend. As the patient's treatment progresses and additional information is attained, e.g., actual hemoglobin measurements, the model is adapted to the individual patient. In particular, the model can be fitted to a particular patient's data, including the patient's endogenous erythropoietin (EPO) levels and the patient's bone marrow reserves. Additional data that may be utilized in the model, include, and are not limited to: red blood cell age distribution; chemotherapy type and dose; chemotherapy history of the patient; patient's sex, age, height, and weight; patient's general health condition; and any "clinical events" that may occur during chemotherapy treatment, e.g., fever. The predicted hemoglobin trend from one or more proposed medical treatments can be displayed for viewing by the physician and/or patient. Each proposed treatment may then be placed into a context to facilitate an understanding of the risk-benefit profile associated with each treatment.

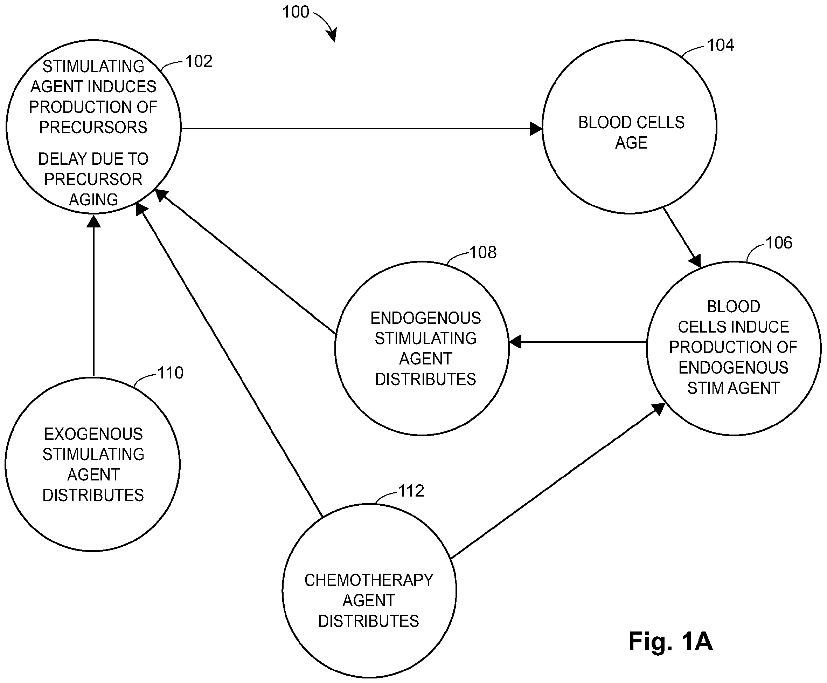

An overview of one embodiment of the mathematical model(s) utilized by the system and method of the embodiments described herein is illustrated in a diagram 100 shown in Fig. 1A. The diagram 100 depicts feedback to achieve homeostasis in erythropoiesis as well as the effects of erythropoiesis stimulating agents (ESAs) and chemotherapy. The body's maturation of red blood cell precursors and the age distribution of these precursors are modeled 102 by the system and method. The production of precursors may be induced by the body's endogenous erythropoietin (EPO), as well as administered exogenous erythropoiesis stimulating agents (ESA), e.g., but not limited to, Aranesp^{®}, Epogen^{®}, Procrit^{®} or biosimilars thereof. The red blood cell precursors reside in the bone marrow and will typically eventually mature into red blood cells. The system and method model 104 the ever changing age distribution of the red blood cells within the body's blood stream. When there are not enough red blood cells in the blood stream, the body increases the rate of production of erythropoietin, which is needed to stimulate stem cells to produce more red blood cell precursors. The body's process for inducing production of erythropoietin is modeled 106 by the system and method. The system and method further model 108 the distribution of the erythropoietin. In addition, the system and method are capable of modeling multiple agents utilized to affect multiple cell population, such as a dosing of an erythropoiesis stimulating agent 110 and a distribution of a chemotherapy agent 112 into the body.

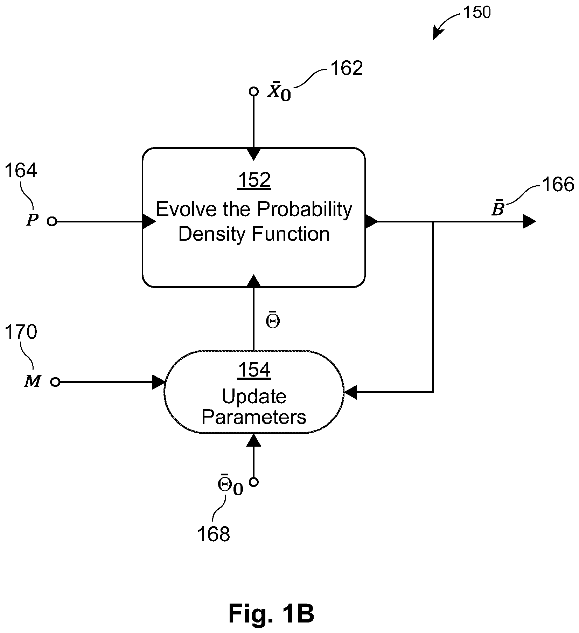

Figure 1B is a flow diagram for adapting a generalized model 150 to an individual patient. The model 150 may be any one of the models 102-112, and/or any combination thereof, as described with respect to Fig. 1A. It should be appreciated that the model 150 may include both parameters associated with the patient that are known (such as gender, age, blood type, age range, etc.), as well as parameters associated with the patient that are difficult to measure and/or characterize. Accordingly, the model 150 may include a probability density function (PDF) 164 (illustrated as P) to model the potential variances for the latter type of parameters (illustrated as 0). In these embodiments, because the model 150 includes a PDF function, the output of the model for any particular parameter may also be represented as a PDF function. In the aforementioned erythropoiesis system, the parameters modeled by the PDF 164 may include sensitivity to ESAs, distributivity of dosages, a rate at which blood cells age, and so on.

The PDF 164 may not generate a single value, but, rather, a probability that the value falls within a particular range of values. Typically, the mean, median, or mode of the PDF 164 is utilized to convert the output 166 (illustrated as *B̃*) of the PDF 164 to a value that may be utilized in non-heuristic systems and/or models. That said, other techniques may be utilized to convert the PDF 164 into a suitable output 166 for the model 150.

In some embodiments, the model 150 may be adapted over time using a tool for Bayesian prediction and fitting of stochastic models, such as Markov models. For example, the tool may utilize particle filtering techniques. In particle filtering, each state and the corresponding set of parameter values is referred to as a "particle" (or, sometimes, a "sample"). Each of these particles may then be input into the model 150 to determine a predictive output of the model 150. Accordingly, based on the current state of the model 150, the methods and systems may involve selecting a particular particle to utilize as an input to a subsequent state of the model 150. That said, other tools for Bayesian prediction and fitting of stochastic models may also be utilized, for example adaptive mixture filtering, Kalman filtering, grid-based methods, and so on. Accordingly, any reference to particle filtering techniques is non-limiting and envisions the alternative use of another tool for Bayesian prediction and fitting of stochastic models.

As illustrated, the flow diagram may begin with an initial state 162 of the model 150 (illustrated as *X̃₀*) and an initial set of parameters 168 modeled by the PDF 164 (illustrated as *Θ̃*₀). The initial state 162 may be the generalized model for the system that represents the modeled behavior for an entire population. Similarly, the generalized model may provide the initial set of parameters 168. In some embodiments, the known parameters associated with the patient may be input into the model 150.

At block 152, one or more processors may evolve the PDF 164. Evolving the PDF 164 may include incrementing a time parameter to cause the model 150 to enter the next state. To this end, the PDF 164 may utilize the incremented time parameter, as well as the set of parameters 8 to generate a new output 166. In some embodiments, for each output parameter of the model 150, the output 166 may be the value (or particle) associated with the median marginal density for the PDF function corresponding to the parameter.

At block 154, the one or more processors may update the parameters *Θ̃* associated with the model 150. In scenarios where there is no new measurement 170 (illustrated as M), the parameters included in the output 166 may be used as the set of parameters 8 in the next state of the model 150. On the other hand, if there is a new measurement 170 (such as a new hemoglobin measurement), the set of parameters included in the output 166 may be modified based on the measurement 170. As described above, due the high variances associated with the model 150 at the onset, the model 150 initially may be relatively inaccurate for modeling a response for an individual patient. Accordingly, the measurement(s) 170 enable model 150 to narrow the variances of the model 150 around the region associated with the measured values. Said another way, the width of the PDF functions associated with the parameters *Θ̃* may be reduced. Thus, over time, the model 150 is adapted to be tailored the patient's measured responses.

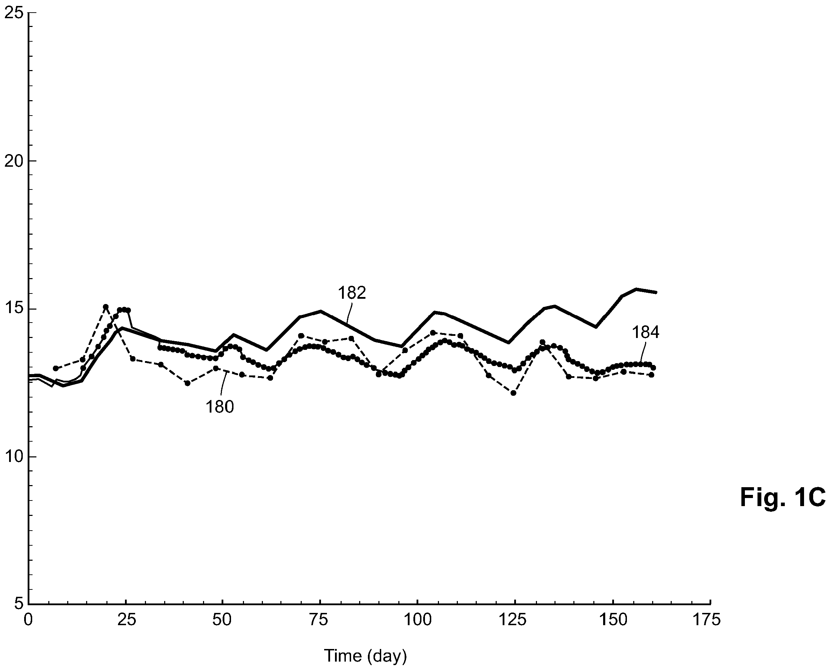

It should be appreciated that this shift in the model 150 can be manifested graphically. Figure 1C is a graphical representation 175 that depicts the improvement in prediction provided by the present adaptation techniques. The graph 175 depicts the patient's measured hemoglobin levels 180 over time, as well as the predicted hemoglobin levels produced by the generalized model without adaptation 182 and the predicted hemoglobin levels produced by the model adapted utilizing the present techniques 184.

As illustrated, the predicted hemoglobin levels 182 and 184 both are relatively accurate at the onset of the prediction horizon. Over time, the predicted hemoglobin levels produced from the generalized model 184 begin to drift further away from the measured hemoglobin levels 180. Conversely, as more measurements made, the predicted hemoglobin levels from the adapted model 184 track more closely to the measured hemoglobin levels 180. As a result, when setting a treatment strategy for the patient, the predictions relied upon to ensure the patient does not experience a critical hemoglobin are more accurate and can reduce the likelihood that predictive error results in the patient experiencing an unexpected medical event.

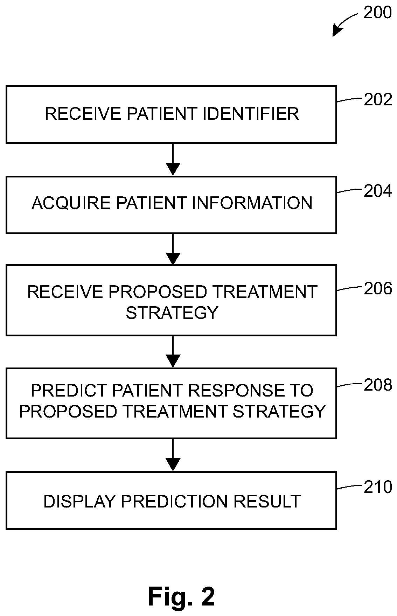

Fig. 2 is a flow diagram of an example method 200 capable of being implemented by an embodiment of the system and method described herein. The method 200 is directed to treating a patient with a medical condition related to an undesirable occurrence in at least one population of *in vivo* cells. Generally speaking, the method 200 applies an adaptive dynamically predictive model to information associated with the patient, wherein the patient is treated based on the output predicted by the predictive model. In one embodiment, the method 200 is used by medical personnel to treat a cancer patient at risk of developing chemotherapy induced anemia (CIA). The patient is exclusively associated with an identifier, which may be newly generated or already exist (block 202), e.g., social security number; and is used to access information about the patient (block 204). The patient information may be acquired from one or more devices and be in one or more types of format, e.g., electronic and/or non-electronic format. For example, the device may be an electronic medical record (EMR), patient record, (EPR), health record (EHR), or personal health record (PHR), and the patient information may be stored on a single storage device or stored in several storage devices distributed in different locations. The device may also be a sensor capable of being operatively coupled to the patient and the system, wherein current or stored patient information acquired and/or stored within the sensor and/or data memory may be accessed by the system and method. The patient information includes values, or clinical values, related to clinical events associated with the patient. Clinical events may include, and are not limited to, tests, treatments, measurements, and assessments associated with the patient. The clinical values may include biological and/or physiological information and/or characteristics, such as: measured hemoglobin levels; red blood cell age distribution; chemotherapy history (type and dose); sex, age, height, and weight; general health condition; and any clinical "events" that may occur during a medical treatment, e.g., test results, prescriptions, administered medicaments, patient condition.

Medical personnel, e.g., a physician, may utilize the patient information to develop and or propose a treatment strategy for the patient. The proposed treatment strategy may include one or more separate or combinable strategies. The treatment strategy may include an action that is performed on, or with, the patient as well as an inaction that is not performed on, or with, the patient, such as waiting a determined period of time. Some examples of a treatment strategy include, and are not limited to, watchful waiting (and consideration of further treatment at a later time); watchful waiting and transfusion of the patient's blood if the hemoglobin concentration level is below 8 g/dL; and dosing a drug according to its label.

The treatment strategy of watchful waiting may primarily include "watching" the patient for a period of time. In other words, the patient may be monitored by medical personnel and continue whatever treatment may currently be prescribed, if any, and then record and/or report the patient's condition, e.g., signs of symptoms. For example, periodically checking the patient's hemoglobin concentration for a predetermined time. The treatment strategy of watchful waiting and transfusion of the patient's blood if the hemoglobin concentration is below 8 g/dL requires monitoring the patient's hemoglobin level for a predetermined time, and if patient's hemoglobin concentration falls below 8 g/dL, initiate a blood transfusion on the patient. The treatment strategy of dosing a drug, e.g., erythropoiesis stimulating agents (ESAs), according to a label requires that the drug be administered to the patient in accordance with the drug's label that has been approved by a regulatory agency or administration, e.g., U.S. Food and Drug Administration. For example, a label for a drug used to treat anemia may require that the drug be used at its lowest dose and only when the patient's hemoglobin concentration is below 10 g/dL.

The proposed treatment strategy is submitted or provided to the system (block 206) and the system predicts the patient's likely response or reaction to the treatment strategy (block 208). For example, the system will determine how the proposed treatment strategy will likely affect the patient's hemoglobin concentration level. If the prediction is conducted early in the treatment of the patient, such that patient information may be limited, the calculation will be based on statistical averages of other individuals who have the same or similar characteristics of the particular patient. e.g., a cohort-based prediction facilitated by machine-learning analyses. As more patient information becomes attainable, the prediction can be adapted to the particular patient. As described with respect to Fig. 1B, the measured values of the patient's clinical events can be used to adapt the corresponding clinical event parameter of the predictive model implemented by the system to fit the model to the patient.

Alternatively, or in addition to the treatment strategies proposed by the physician, the system and method of an embodiment described herein may provide or suggest one or more proposed treatment strategies to the physician for selection. The treatment strategies provided by the system and method may be in response to queries or information presented by or received from treatment personnel. For example, a physician may request the latest date to dose according to the label and maintain the patient's hemoglobin concentration above a specified level. In one embodiment of the system and method described herein, a model predictive controller may use the predicted results to compute a possible dosing that will lead to a desired outcome.

The prediction result is displayed by the system and method (block 210) and may also be stored with the proposed treatment strategy on a memory device for later recall and implementation. The prediction result may include a time profile of the patient's likely response and may be presented in any desired format. The time profile may include time-related information of the patient's expected response to the proposed treatment strategy and in one example, the time profile includes, at least: a line representing an expected trend of the patient's hemoglobin concentration; proposed treatments, e.g., dosing, blood transfusion; and critical hemoglobin levels. The system and method of embodiments described herein are capable of simultaneously displaying one or more time profiles to facilitate comparison between the prediction results of multiple proposed treatment strategies. The physician and/or patient may select to display the time profiles side-by-side, overlapping, in different colors or shapes, etc.

The displayed time profile provides an image to the physician and/or patient that may contribute to a better understanding of the proposed treatment strategy itself, and in relation to one or more other proposed treatment strategies. For example, the times when a dosing or transfusion may be necessary or when the patient may more likely become symptomatic, can be readily observed on the time profile. By visually comparing one or more time profiles, the physician and/or the patient may be better able to identify and/or determine a more favorable treatment strategy.

Additional proposed treatment strategies may later be provided to the system that are continuations of earlier or other previous treatment strategies. For example, additional information about the patient may be attained and adapted to the model for predicting the patient's response to a proposed treatment strategy. The additional information may include values for clinical events associated with the patient, such as biological or physiological information and/or characteristics that were not available earlier, e.g., hemoglobin measurements and/or chemotherapy treatments since the last proposed treatment strategy was evaluated or prescribed. The patient's clinical values may be adapted to respective clinical event parameters that are incorporated in the predictive module implemented by embodiments of the system and method described herein.

A treatment strategy may also be adapted or modified by changing one or more aspects of a predictive result. In particular, the display of the time profile may be modified by selecting an aspect of the display and changing it. For example, an aspect such as a dosing date appearing on a touch-screen display may be selected by medical personnel and moved to a different date or deleted, and/or another aspect may be added to and/or removed from the time profile, e.g., a chemotherapy treatment, a blood transfusion, or a dosing event. After modifications or to the displayed time profile are complete, the modified time profile may be considered a "new" proposed treatment strategy, and a request to generate a prediction of the patient's response to the modified proposed treatment strategy may then be submitted to the system.

It is to be understood that if the proposed treatment includes a dosing that violates the medicament's label, or the predictive result of a proposed treatment strategy includes a dosing a medicament that violates its label, a notification can be provided advising of the violation and the execution to predict the patient's response to the prohibited proposed treatment strategy may not be initiated and/or the associated prediction results, e.g., time profile, may not be displayed.

According to certain aspects, in addition to predicting a patient's response to a treatment strategy, measured data from the patient may be forensically analyzed to determine an effectiveness of the predictive module. In one example, the predictive module, as adapted to the particular patient, is applied to a set of patient data observed at the onset of a treatment strategy. From this historical state, a traditional treatment strategy may be input into the system to predict how the patient would have responded in a traditional scenario. In some scenarios where the present systems and methods generated a treatment strategy that avoided a transfusion, applying the predictive module to the set of data at the onset indicates that if the patient had been treated according to a traditional treatment strategy, the patient would have required a transfusion. Similarly, this technique may be utilized to develop other outcome measurements, such as a reduction in the number of dosages, increasing the amount of time a patient experiences a healthy level of hemoglobin, avoidance of chemotherapy events, and so on. Of course, this technique may be applied across multiple patients to generate statistics that prove the effectiveness of the present systems and methods.

In a further aspect, this forensic analysis may be utilized to improve the predictive module itself. For example, forensic analysis may be utilized to more accurately determine the impact of patient characteristics, such as age or weight, on the developed models. To this end, the forensic analysis may include applying clustering techniques to a pool of anonymized treatment data to determine the impact of treatment events for patients that share common characteristics. Accordingly, when the known patient characteristics are input into the generalized model, the corresponding parameters more closely model the patient from the onset. Said another way, the *Θ̃*₀ used at the start of the modeling process more accurately models the patient. It should be appreciated that even with these improvements, the generalized model may still be improved by adaptation based on the patient's observed responses.

An example chart 300 that may be used by medical personnel to monitor the hemoglobin concentration of an example patient during chemotherapy is shown in Fig. 3. The chart 300 includes an X-axis 302 representing time (days) and a Y-axis 304 representing a hemoglobin concentration (g/dL) of the patient. Completed and/or scheduled chemotherapy cycles 306 administered to the patient are shown by the bands of three vertical lines (in this example, the chemotherapy agent is given every day for three days per cycle, hence the three lines) and instances of measured hemoglobin concentrations are shown by circular markers 308 (e.g., tracked on a weekly basis). Two hemoglobin concentration levels of particular interest are 8 g/dL and 10 g/dL, and each is shown by a horizontal dashed line 310, 312, respectively.

Typical patient treatment strategies implemented in the past included watchful waiting where the physician monitored and tracked the hemoglobin concentration of the patient and the patient's symptoms. To decide further treatment, the physician reacted to the trend of the patient's tracked hemoglobin measurements, as well as the patient's symptoms, and then decided whether and how to treat the patient. The physician essentially reacted to past hemoglobin measurements of the patient and the patient's past and/or current symptoms. Rather than merely reacting to a patient's past hemoglobin measurement(s) and symptoms, a physician utilizing an embodiment of the system and method described herein is able to proactively treat the patient. That is, a physician is able to predicts a patient's likely behavior to a proposed treatment strategy and thus prescribe a treatment strategy for the patient accordingly in an effort to attain a desired result, and/or modify a proposed treatment strategy to likely attain a result different than initially predicted.

Example charts 400, 400', and 400" respectively depicted in Figs. 4A, 4B, and 4C are similar to the chart 300 shown in Fig. 3. However, the charts 400, 400', and 400" in Figs. 4A, 4B, and 4C each include a predicted result respectively associated with a distinctive proposed patient treatment strategy, which is capable of being calculated by the system and method described herein. In each instance, the proposed treatment strategy may have been presented to and/or provided by the physician via the system's user interface. A prediction module, when executed by one or more processors of the system, generates the predicted result of the proposed treatment strategy. The predicted result includes a time profile of the patient's likely response to the proposed treatment strategy and is capable of being stored on the system and displayed on the system's user interface. The prediction module may be stored in the system's memory and include one or more models for modeling homeostasis. The prediction module may also be referred to herein as the module, mathematical module, and/or predictive module.

In Fig. 4A, the proposed treatment strategy employs watchful waiting and consideration of additional treatment later on. The system utilizes the available patient information and the predictive model to provide a time profile 414 including a relatively thick solid line representing the patient's expected trend of hemoglobin level 416 over a period of time. The thinner, lighter shade, "gray" line(s) 418 proximate the predicted trend line 416 represent prediction errors that may occur due to the sample distribution, which may be attained by sampling the distribution multiple times and determining an error band.

Further observation of the chart 400 in Fig. 4A shows the predicted trend 416 of the patient's hemoglobin concentration will drop below 8 g/dL 410 at around 100 days. It is likely that the physician would prescribe a blood transfusion at around this time. If a dosing (not shown in Fig. 4A) or a blood transfusion (not shown in Fig. 4A) was proposed by the system in the predicted result, the amount and time of the dosing and/or blood transfusion would be presented on the chart. A predicted dosing is represented by a triangular marker aligned with a day along the horizontal axis 402 and a quantitative amount (e.g., mcg) along a vertical axis 424 positioned on the right side of the chart 400. A blood transfusion is represented by a vertical line that generally coincides with a vertical segment of the projected hemoglobin trend 416, which may appear as having two seemingly separate and disconnected sections. The vertical rise in the hemoglobin trend line is indicative of the immediate effect that occurs to the hemoglobin concentration in response to the blood transfusion.

As described above, it is to be understood that the system and method of the embodiments described herein may be performed at any time during a patient's treatment, e.g., before some or any hemoglobin measurements were taken. The system's prediction of the patient's hemoglobin trend in such an instance would be based on statistical averages of other individuals who have the same or similar characteristics of the particular patient, e.g., a cohort-based prediction. As more data of the individual patient is acquired, the predictive model is able to adapt or "personalize" the hemoglobin trend prediction toward the particular patient. The system is able to adapt the proposed treatment strategy to the patient because the chemotherapy acts like an impulse to the system, and the parameters of the predicted result may therefore be attained or characterized more readily than otherwise.

In the chart 400' of Fig. 4B, the predicted result is in response to a proposed treatment strategy that includes watchful waiting and transfusing if the patient's hemoglobin level drops below 8 g/dL. The predicted trend 416' of the patient's hemoglobin concentration level is shown to drop below 8 g/dL 410' a few days after 100 days, wherein the time profile 414' includes the blood transfusion 420'. The physician and/or the system may have provided a specific amount of blood to be transfused, e.g., 1 or more units(s) (bag(s)) into the predictive model, which may appear in the time profile 414'. Alternatively, the physician may have queried the predictive model as to the amount of blood required for the transfusion to bring the patient's hemoglobin concentration to a desired level, e.g., above 9g/dL.

The predictive effect of a transfusion takes into consideration that the blood provided during the transfusion likely has a shorter "life span" than blood generated by the body. In some instances the life span of the transfused blood may be estimated to be 50 days, or perhaps as short as 30 days or less. The transfusion provides an immediate augmentation of red blood cells into the patient's circulatory system, which can be easily observed in the time profile displayed on the chart. Since the red blood cell age population distribution is being tracked in the model(s), the predictive model takes into consideration how quickly the effect of the transfusion may dissipate. Thus, it is important to accurately predict the magnitude of the increase in the patient's hemoglobin level. Other consequences that may be considered by the predictive model concern iron concentration that results when iron is released into the body when the hemoglobin expires.

In the chart 400" of Fig. 4C, the predicted result is in response to a proposed treatment strategy that includes dosing an erythropoiesis stimulating agent (ESA), e.g., Aranesp^{®}, according to the label after two rounds of chemotherapy. The system may have provided a specific amount and time for the dosing or the physician may have queried the predictive model as to the time and amount of dosing on label to maintain the patient's hemoglobin above a specified level, e.g., above 8.5 g/dL. The predicted dosings 422" are represented by a triangular marker aligned with a day along the horizontal axis 402". The quantitative amount (e.g., mcg) of the dosing 422" is readily observable by the alignment of the triangular marker with the vertical axis 424" positioned on the right side of the chart.

Medical personnel and/or the patient may use the charts 400, 400', and 400" in Figs. 4A, 4B, and 4C to compare the risk-benefit between the respective treatment strategies. The amount and timing of the blood transfusion 420' and/or dosing 422" are readily observable on the chart and aspects of the time profiles 416, 416', 416" may also be easily adjusted via a treatment strategy module to modify and/or propose a different treatment strategy. The treatment strategy module may be stored in the system's memory, and when executed on one or more processors, allows a physician to propose changes to the treatment strategy by enabling the user to modify aspects of an available treatment, e.g., watchful waiting, blood transfusion, dosing, hemoglobin concentration level, etc. A prediction module utilizes a selected treatment strategy to predict an outcome including the patient's response to the treatment strategy and displays the calculate result, e.g., time profile, on the system's user interface. One or more predictions, e.g., time profiles, may be arranged for display on the user interface in various configurations, such as side-by-side, overlapping, and in different colors or shapes for evaluation by medical personnel.

The example charts 500, 500', 500" illustrated in Figs 5A, 5B, and 5C, respectively, each include a predictive result for a particular patient in response to a different proposed treatment strategy prescribed after the completion of four cycles of chemotherapy, which may have been presented to and/or provided by the physician via the system's user interface. In Fig. 5A, the proposed treatment strategy includes watchful waiting and a blood transfusion if the patient's hemoglobin level drops below 8 g/dL after two rounds of chemotherapy, and the same proposed treatment strategy after four rounds of chemotherapy. The predicted trend 516 of the patient's hemoglobin concentration level is shown to drop below 8 g/dL a few days after 100 days, wherein the time profile 514 includes the blood transfusion 520. The physician and/or the system may have provided a specific amount of blood to be transfused, e.g., 1 or more unit(s) (bag(s)) into the predictive model. Alternatively, the physician may have queried the predictive model as to the amount of blood required for the transfusion to bring the patient's hemoglobin concentration to a desired level, e.g., above 9 g/dL.

In Fig. 5B, the chart 500' of the predictive results displays a time profile 514' including a predictive trend 516' of the particular patient's hemoglobin level in response to a proposed treatment strategy, which may have been presented to, or provided by, the physician. In this instance, the proposed treatment strategy includes watchful waiting and a blood transfusion if the hemoglobin level drops below 8 g/dL after two rounds of chemotherapy, and dosing an erythropoiesis stimulating agent (ESA), e.g., Aranesp^{®}, according to the label after four rounds of chemotherapy. The predicted trend 516' of the patient's hemoglobin concentration is shown to drop below 8 g/dL 510; a few days after initiation of the dosing 522' and the completion of the fifth therapy cycle. The system may have provided a specific amount and time for the dosing or the physician may have queried the predictive model via the system's user interface as to the time and amount of dosing on label to maintain the patient's hemoglobin above a specified level, e.g., above 8.0 g/dL. The predicted dosings 520' are represented by a triangular marker aligned with a day along the horizontal axis. The quantitative amount (e.g., mcg) of the dosing 520' is readily observable by the alignment of the triangular marker with the vertical axis 524' positioned on the right side of the chart 500'.

In Fig. 5C, the chart 500" of the predictive results displays a time profile 514" including a predictive trend 516" of the particular patient's hemoglobin concentration level in response to a proposed treatment strategy, which may have been presented to and/or provided by the physician via the system's user interface. In this instance, the proposed treatment strategy includes watchful waiting and transfusion if the patient's hemoglobin level drops below 8 g/dL after two rounds of chemotherapy, and dosing an erythropoiesis stimulating agent (ESA), e.g., Aranesp^{®}, according to the label after four rounds of chemotherapy. The predicted dosing 522' is shown to begin just after the completion of the fifth chemotherapy cycle. However, due to the delayed effect of the drug therapy, the predicted trend 516' of the patient's hemoglobin concentration level is shown to drop below 8 g/dL 510" after dosing 522" has begun. Some days thereafter, a predicted near immediate increase in the hemoglobin concentration level is shown by the expected upward spike in the hemoglobin concentration trend 516" resulting from the blood transfusion 520".

Medical personnel and/or the patient may use the respective charts 500, 500', 500" in Figs. 5A, 5B, and 5C to analyze the risk-benefit of each proposed treatment strategy. In the proposed transfusion treatment shown in Fig. 5A, an immediate increase to the patient's hemoglobin concentration 516 in response to the transfusion 520 can be observed. In the proposed dosing treatment 522' shown in Fig. 5B, a continued decrease in the predicted hemoglobin trend 516' is shown to drop below the 8 g/dL level, followed by a steeper decrease in the hemoglobin level, before the hemoglobin level begins to increase. In Fig. 5C, the "synergistic" effect resulting from the timing and amount of the dosing 522" and the blood transfusion 520" may generally be considered an inefficient management of the patient's treatment and that such a proposed treatment strategy may yield less than favorable results.

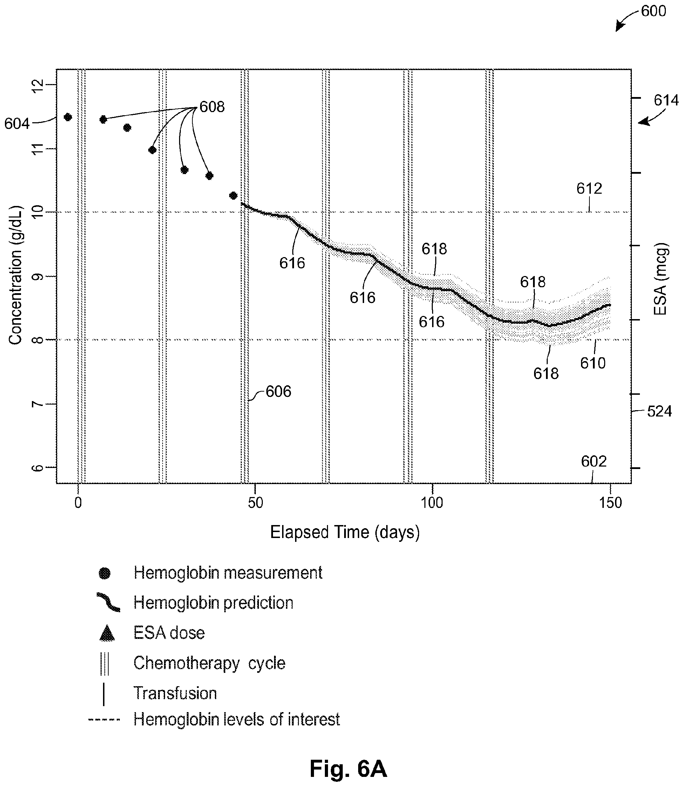

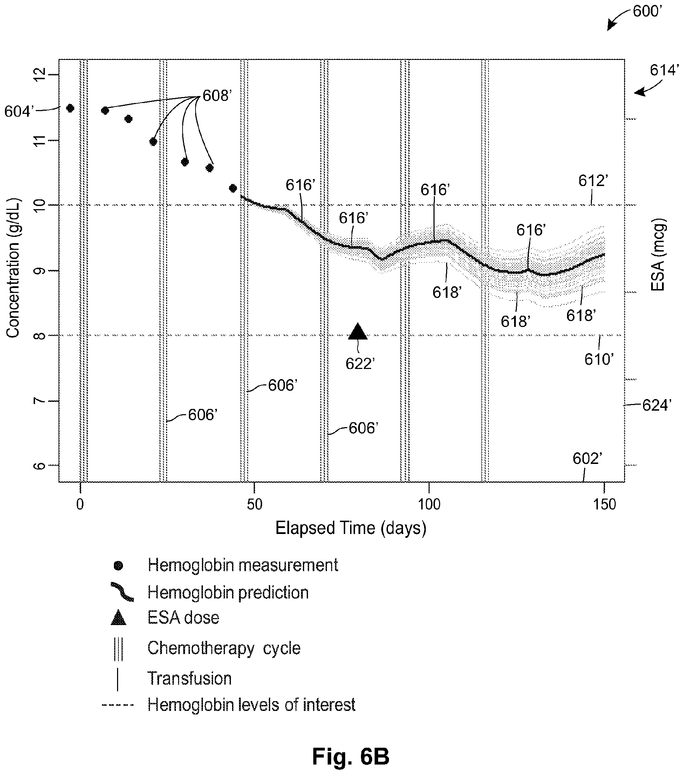

Figs. 6A and 6B respectively illustrate a chart 600, 600', respectively, including a predictive result in response to a prescribed treatment strategy proposed for a particular patient after the completion of two cycles of chemotherapy. The treatment strategy may have been presented to and/or provided by the physician via the system's user interface. In Fig. 6A, the proposed treatment strategy includes watchful waiting and consideration of later treatment. The example chart 600 of the predictive results displays a time profile 614 including a predictive trend 616 of the patient's hemoglobin concentration level in response to the proposed treatment strategy. The predictive model did not predict a dosing or a transfusion of the patient. The physician may monitor the patient and if the patient has no symptoms, the patient may be able to complete chemotherapy without a dosing and/or a blood transfusion.

In Fig. 6B, the proposed treatment strategy includes watchful waiting and consideration of later treatment after another two rounds of chemotherapy, and dosing an ESA according to the label after four rounds of chemotherapy. The example chart 600' of the predictive results displays a time profile 614' including a predictive trend 616' of the patient's hemoglobin concentration level in response to the proposed treatment strategy. In this instance, the system predicted a projected dosing 622' time and amount and the projected trend 616' of the hemoglobin level. Providing a single ESA dose 622' in this case may help the patient not develop symptoms that may occur as the hemoglobin concentration trends toward 8 g/dL, which also risks the need for a blood transfusion if the hemoglobin concentration should fall below 8 g/dL 610'. By prescribing a single dose 622' as predicted in Fig. 6B, the patient's hemoglobin concentration level is more likely to stay above the 8 g/dL level 610' and the patient is less likely to develop symptoms. The physician as well as the patient can readily observe this information on the display. With further inspection of the predicted hemoglobin trend 616' and the prescribed dosing 622', it can be seen that a few days after dosing 622', the patient's hemoglobin concentration level 616' continues to descend and then there is a reversal and the patient's hemoglobin concentration 616' begins to increase. This increase in the hemoglobin concentration level 616' may reflect the body's response to sensing the decrease in hemoglobin concentration level and producing more endogenous EPO to raise the hemoglobin concentration level. The degree to which the predictive trend increases indicates how sensitive the body is to its own endogenous EPO. Through modeling of homeostasis, an understanding of the effectiveness of an erythropoiesis stimulating agent as compared to the effectiveness of the body's endogenous EPO can be attained. With this understanding, the effect from dosing an erythropoiesis stimulating agent may be calculated by the predictive model utilized in an embodiment of the method and system described herein.

Some sources estimate that approximately one-third of patients recovering from chemotherapy treatment receive two or more blood transfusions. The charts illustrated in Figs 7A, 7B, and 7C each include a predictive result for an example patient in response to a proposed treatment strategy prescribed after the completion of two cycles of chemotherapy. The treatment strategy may have been presented to and/or provided by the physician via the system's user interface. In Fig. 7A, the example chart 700 illustrates a predictive result to the patient's prescribed treatment strategy of watchful waiting and consideration of further treatment after two rounds of chemotherapy. This patient appears very sensitive to chemotherapy as alluded to by the system's predicted time profile 714 and the hemoglobin concentration trend 716 showing that two transfusions 720 are likely to be needed during the chemotherapy treatment.

In Fig. 7B, an example chart 700' of the displayed predictive result shows a time profile 714' including a predictive trend 716'of the patient's hemoglobin concentration in response to another proposed treatment strategy including dosing an erythropoiesis stimulating agent (ESA) according to the label after two rounds of chemotherapy and then watchful waiting with consideration of later treatment thereafter. The system predicts that the first transfusion 720 likely needed in Fig. 7A can be avoided by two dosings 722' of ESA according to the label.

In Fig. 7C, the predictive result for another proposed treatment strategy for the example patient is illustrated in example chart 700". The proposed treatment strategy includes dosing according to the label throughout the chemotherapy treatment. The system predicts that the patient can avoid the first and second transfusions 720 shown in Fig. 1 by prescribing four doses 722" of ESA according to the label.

Medical personnel and/or the patient may use the respective charts 700, 700', 700" in Figs. 7A, 7B, and 7C to analyze the risk-benefit of each proposed treatment strategy. For example, in the proposed transfusion treatment shown in Fig. 7C, four proposed dosings of an ESA according to the label may avert the need for the blood transfusions suggested as necessary in the prescribed treatment strategies of Figs. 7A and 7B.

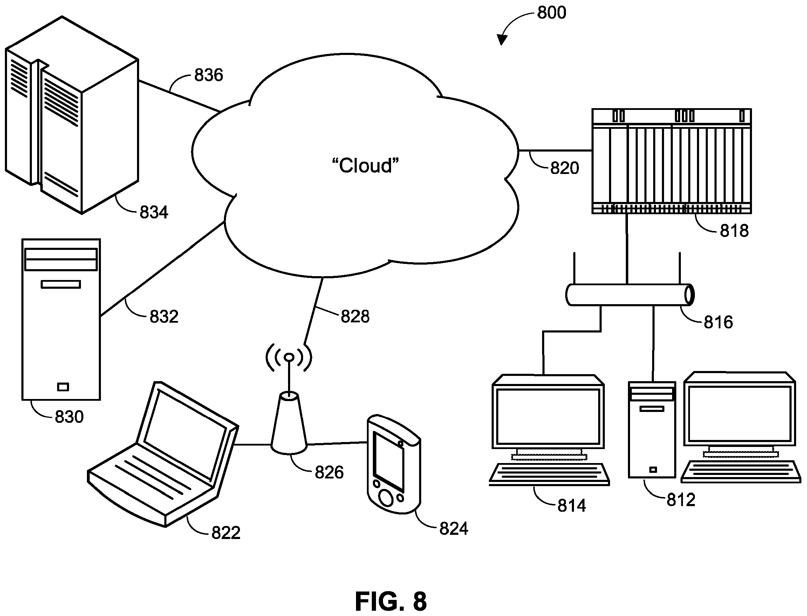

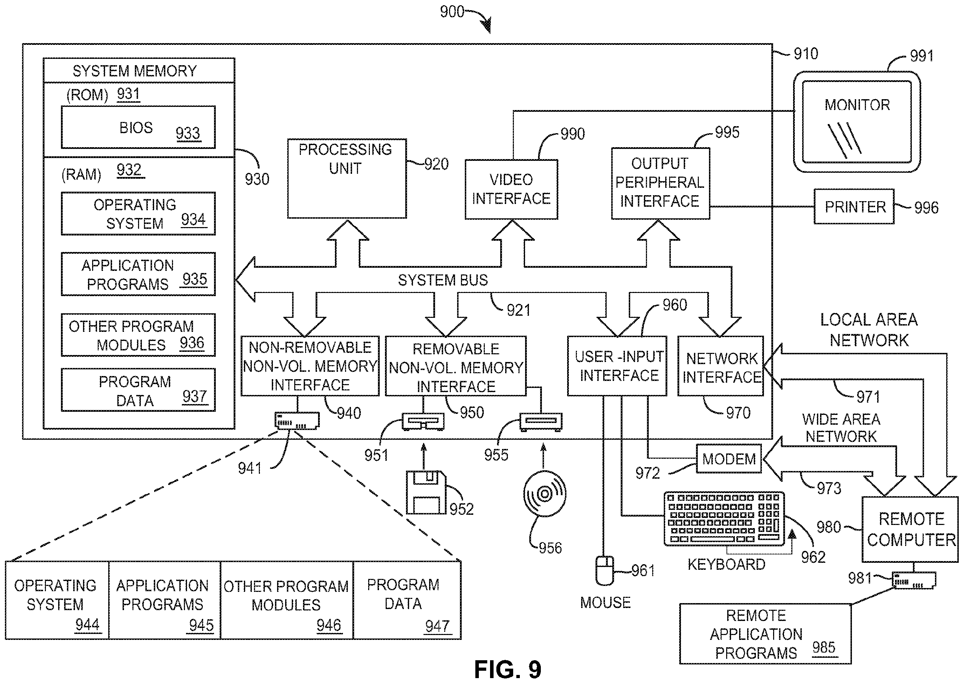

The system and method for treating a patient incorporated by the embodiments described herein may be implemented using an electronic computing system. Figs. 8 and 9 provide examples of a structural basis for the network and computational platforms related to such an electronic computing system.

Fig. 8 illustrates an exemplary block diagram of a network 800 and computer hardware that may be utilized in an exemplary system for treating a patient in accordance with the described embodiments. The network 800 may be the internet, a virtual private network (VPN), or any other network that allows one or more computers, communication devices, databases, etc., to be communicatively connected to each other. The network 800 may be connected to a personal computer 812, and a computer terminal 814 via an Ethernet 816 and a router 818, and a landline 820. The Ethernet 816 may be a subnet of a larger Internet Protocol network. Other networked resources, such as projectors or printers (not depicted), may also be supported via the Ethernet 816 or another data network. Additionally, the network 800 may be wirelessly connected to a laptop computer 822 and a personal data assistant 824 via a wireless communication station 826 and a wireless link 828. Similarly, a server 830 may be connected to the network 800 using a communication link 832 and a mainframe 834 may be connected to the network 800 using another communication link 836. The network 800 may be useful for supporting peer-to-peer network traffic. Patient information, e.g., EMR, may also be received from a remotely-accessible, free-standing memory device (not shown) on the network 800. In some embodiments, the patient information may be received by more than one computer. In other embodiments, the patient information may be received from more than one computer and/or remotely-accessible memory device.

Some or all calculations performed in the determination of a patient's response to proposed treatment strategies may be performed by one or more computing devices such as the personal computer 812, laptop computer 822, server 830, and/or mainframe 834, for example. In some embodiments, some or all of the calculations may be performed by more than one computer.

Providing a time profile of the predicted results attained by the embodiment described herein may also be performed by one or more computing devices as the personal computer 812, laptop computer 822, server 830, and/or mainframe 834, for example. In some embodiments, displaying the predicted results, e.g., hemoglobin trend, may include sending data over a network such as network 800 to another computing device or display device.

Fig. 9 illustrates an exemplary block diagram of a system 900 on which an exemplary method for treating a patient may operate in accordance with the described embodiments. The system 900 of Fig. 9 includes a computing device in the form of a computer 910. Components of the computer 910 may include, and are not limited to, a processing unit 920, a system memory 930, and a system bus 921 that couples various system components including the system memory to the processing unit 920. The system bus 921 may be any of several types of bus structures including a memory bus or memory controller, a peripheral bus, and a local bus using any of a variety of bus architectures. By way of example, and not limitation, such architectures include the Industry Standard Architecture (ISA) bus, Micro Channel Architecture (MCA) bus, Enhanced ISA (EISA) bus, Video Electronics Standards Association (VESA) local bus, and Peripheral Component Interconnect (PCI) bus (also known as Mezzanine bus).

The computer 910 typically includes a variety of computer readable media. Computer readable media can be any available media that can be accessed by the computer 910 and may include volatile and/or nonvolatile media, as well as removable and/or non-removable media. By way of example, and not limitation, computer readable media may comprise computer storage media and communication media. Computer storage media includes volatile and nonvolatile, removable and non-removable media implemented in any method or technology for storage of information such as computer readable instructions, data structures, program modules or other data. Computer storage media includes, but is not limited to, RAM, ROM, EEPROM, FLASH memory or other memory technology, CD-ROM, digital versatile disks (DVD) or other optical disk storage, magnetic cassettes, magnetic tape, magnetic disk storage or other magnetic storage devices, or any other medium which can be used to store the desired information and which can be accessed by computer 910. Communication media typically embodies computer readable instructions, data structures, program modules, or other data in a modulated data signal such as a carrier wave or other transport mechanism and includes any information delivery media. The term "modulated data signal" means a signal that has one or more of its characteristics set or changed in such a manner as to encode information in the signal. By way of example, and not limitation, communication media includes wired media such as a wired network or direct-wired connection, and wireless media such as acoustic, radio frequency (RF), infrared, and other wireless media. Combinations of any of the above are also included within the scope of computer readable media.

The system memory 930 includes computer storage media in the form of volatile and/or nonvolatile memory such as read only memory (ROM) 931 and random access memory (RAM) 932. A basic input/output system 933 (BIOS), containing the basic routines that help to transfer information between elements within computer 910, such as during start-up, is typically stored in ROM 931. RAM 932 typically contains data and/or program modules or routines, e.g., analyzing, calculating, predicting, indicating, determining, etc., that are immediately accessible to and/or presently being operated on by a processing unit 920, e.g., prediction module, treatment strategy module. By way of example, and not limitation, Fig. 9 illustrates operating system 934, application programs 935, other program modules 936, and program data 937.

The computer 910 may also include other removable/non-removable, volatile/nonvolatile computer storage media. By way of example only, Fig. 9 illustrates a hard disk drive 941 that reads from or writes to non-removable, nonvolatile magnetic media, a magnetic disk drive 951 that reads from or writes to a removable, nonvolatile magnetic disk 952, and an optical disk drive 955 that reads from or writes to a removable, nonvolatile optical disk 956 such as a CD ROM or other optical media. Other removable/non-removable, volatile/nonvolatile computer storage media that can be used in the exemplary operating environment include, but are not limited to, magnetic tape cassettes, flash memory cards, digital versatile disks, digital video tape, solid state RAM, solid state ROM, and the like. The hard disk drive 941 is typically connected to the system bus 921 through a non-removable memory interface such as interface 940, and magnetic disk drive 951 and optical disk drive 955 are typically connected to the system bus 921 by a removable memory interface, such as interface 950.

The drives and their associated computer storage media discussed above and illustrated in Fig. 9 provide storage of computer readable instructions, data structures, program modules and other data for the computer 910. In Fig. 9, for example, hard disk drive 941 is illustrated as storing operating system 944, application programs 945, other program modules 946, and program data 947. Note that these components can either be the same as or different from operating system 934, application programs 935, other program modules 936, and program data 937. Operating system 944, application programs 945, other program modules 946, and program data 947 are given different numbers here to illustrate that, at a minimum, they are different copies. A user may enter commands and information into the computer 910 through a user interface module including input devices such as a keyboard 962 and cursor control device 961, commonly referred to as a mouse, trackball, touch-screen, or touch pad. A screen 991 or other type of display device is also connected to the system bus 921 via an interface, such as a graphics controller 990. In addition to the screen 991, computers may also include other peripheral output devices such as printer 996, which may be connected through an output peripheral interface 995.

The computer 910 may operate in a networked environment using logical connections to one or more remote computers, such as a remote computer 980. The remote computer 980 may be an integrated monitoring system operatively coupled to an individual via an input/output component or device, e.g., one or more sensors capable of being connected or attached to the patient and sensing biological and/or physiological information. The logical connections depicted in Fig. 9 include a local area network (LAN) 971 and a wide area network (WAN) 1273, but may also include other networks. Such networking environments are commonplace in hospitals, offices, enterprise-wide computer networks, intranets, and the internet.

When used in a LAN networking environment, the computer 910 is connected to the LAN 971 through a network interface or adapter 970. When used in a WAN networking environment, the computer 910 typically includes a modem 972 or other means for establishing communications over the WAN 973, such as the internet. The modem 972, which may be internal or external, may be connected to the system bus 921 via the input interface 960, or other appropriate mechanism. In a networked environment, program modules depicted relative to the computer 910, or portions thereof, may be stored in the remote memory storage device 981. By way of example, and not limitation, Fig. 9 illustrates remote application programs 985 as residing on memory device 981.

The communications connections 970, 972 allow the device to communicate with other devices. The communications connections 970, 972 are an example of communication media, which may include both storage media and communication media.

The computing 910 may perform the various processing functions described herein in conjunction with the one or more computers 980 or the various functions may be performed solely by the computing device 910. That is, the processing functions performed by the system may be distributed among a plurality of computes configured in an arrangement known as "cloud computing." This arrangement may provide several advantages, such as, for example, enabling near real-time uploads and downloads of information as well as periodic uploads and downloads of information. This arrangement may provide for a thin-client embodiment of a mobile computer or tablet and/or stationary computer described in Fig. 8 as a primary backup of some or all of the data gathered by the one or more computers.

The embodiments for the methods of determining predictive results associated with a patient's response to a proposed treatment strategy described above may be implemented in part or in their entirety using one or more computer systems such as the computer system 900 illustrated in Fig. 9. The patient information and/or predictive models may be received by a computer such as the computer 910, for example. The patient information and/or predictive models may be received over a communication medium such as local area network 971 or wide area network 973, via network interface 970 or user-input interface 960, for example. As another example, the patient information and/or predictive models may be received from a remote source such as the remote computer 980 where the data is initially stored on memory device such as the memory storage device 981. As another example, the patient information and/or predictive models may be received from a removable memory source such as the nonvolatile magnetic disk 952 or the nonvolatile optical disk 956. As another example, the patient information and/or predictive models may be received as a result of a human entering data through a user interface, such as a touch-screen, touch pad, and/or keyboard 962.

Some or all analyzing or calculating performed in the prediction of a patient's response to a proposed treatment strategy described above may be performed by a computer such as the computer 910, and more specifically may be performed by one or more processors, such as the processing unit 920, for example. In some embodiments, some calculations may be performed by a first computer such as the computer 910 while other calculations may be performed by one or more other computers such as the remote computer 980. The analyses and/or calculations may be performed according to instructions that are part of a program such as the application programs 935, the application programs 945 and/or the remote application programs 985, for example.

Predicting a patient's response to a proposed treatment strategy as described above in the embodiments may also be performed by a computer such as the computer 910. The indications may be made by setting the value of a data field stored in the ROM memory 931 and/or the RAM memory 932, for example. In some embodiments, displaying prediction results including the time profile to a user may include sending data over a network such as the local area network 971 or the wide area network 973 to another computer, such as the remote computer 981. In other embodiments, displaying prediction results including the time profile to a user may include sending data over a video interface such as the video interface 990 to display information relating to the prediction on an output device such as the screen 991 or the printer 996, for example.

In certain embodiments described herein, the term "erythropoiesis stimulating agents" ("ESAs") is used. ESAs are pharmaceutical agents that act to stimulate red blood cell production (erythropoiesis) in a subject in need thereof. Nonlimiting examples include Aranesp^{®} (darbepoetin alfa), Epogen^{®} or Procrit^{®} (epoetin alfa), NeoRecormon^{®} (epoetin beta), or Mircera^{®} (methoxy polyethylene glycol-epoetin beta). Additionally, biosimilars of the above innovator ESAs, and additionally other classes of ESAs that may act in a mechanistically different manner (e.g. HIF inhibitors) are also contemplated.

In certain embodiments described herein, the term "chemotherapy" is used. Chemotherapy is a type of cancer treatment that uses chemical substances (chemotherapeutics) to kill or inhibit the undesired growth or overgrowth of cells. Cancer is an example of this undesired growth or overgrowth of cells. Nonlimiting examples of chemotherapeutic agents can include anti-neoplastic agents including, for example, alkylating agents including: nitrogen mustards, such as mechlorethamine, cyclophosphamide, ifosfamide, melphalan and chlorambucil; nitrosoureas, such as carmustine (BCNU), lomustine (CCNU), and semustine (methyl-CCNU); ethylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrimidine analogs such as 5-fluorouracil, fluorodeoxyuridine, gemcitabine, cytosine arabinoside (AraC, cytarabine), 5-azacytidine, 2,2'-difluorodeoxycytidine, purine analogs such as 6-mercaptopurine, 6-thioguanine, azathioprine, 2'-deoxycoformycin (pentostatin), erythrohydroxynonyladenine (EHNA), fludarabine phosphate, and 2-chlorodeoxyadenosine (cladribine, 2-CdA); natural products including antimitotic drugs such as paclitaxel, vinca alkaloids including vinblastine (VLB), vincristine, and vinorelbine, taxotere, estramustine, and estramustine phosphate; epipodophyllotoxins such as etoposide and teniposide; antibiotics such as actimomycin D, daunomycin (rubidomycin), doxorubicin, mitoxantrone, idarubicin, bleomycins, plicamycin (mithramycin), mitomycinC, and actinomycin; enzymes such as L-asparaginase; miscellaneous agents including platinium coordination complexes such as cisplatin and carboplatin, anthracenediones such as mitoxantrone, substituted urea such as hydroxyurea, methylhydrazine derivatives including N-methylhydrazine (MIH) and procarbazine, adrenocortical suppressants such as mitotane (o,p'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide.

Throughout this specification, plural instances may implement components, operations, or structures described as a single instance. Although individual operations of one or more methods are illustrated and described as separate operations, one or more of the individual operations may be performed concurrently, and nothing requires that the operations be performed in the order illustrated. Structures and functionality presented as separate components in example configurations may be implemented as a combined structure or component. Similarly, structures and functionality presented as a single component may be implemented as separate components. These and other variations, modifications, additions, and improvements fall within the scope of the subject matter herein.

Additionally, certain embodiments are described herein as including logic or a number of routines, subroutines, applications, or instructions. These may constitute either software (e.g., code embodied on a machine-readable medium) or hardware. In hardware, the routines, etc., are tangible units capable of performing certain operations and may be configured or arranged in a certain manner. In example embodiments, one or more computer systems (e.g., a standalone, client or server computer system) or one or more hardware modules of a computer system (e.g., a processor or a group of processors) may be configured by software (e.g., an application or application portion) as a hardware module that operates to perform certain operations as described herein.

In various embodiments, a hardware module may be implemented mechanically or electronically. For example, a hardware module may comprise dedicated circuitry or logic that is permanently configured (e.g., as a special-purpose processor, such as a field programmable gate array (FPGA) or an application-specific integrated circuit (ASIC) to perform certain operations. A hardware module may also comprise programmable logic or circuitry (e.g., as encompassed within a general-purpose processor or other programmable processor) that is temporarily configured by software to perform certain operations. It will be appreciated that the decision to implement a hardware module mechanically, in dedicated and permanently configured circuitry, or in temporarily configured circuitry (e.g., configured by software) may be driven by cost and time considerations.

Accordingly, the term "hardware module" should be understood to encompass a tangible entity, be that an entity that is physically constructed, permanently configured (e.g., hardwired), or temporarily configured (e.g., programmed) to operate in a certain manner or to perform certain operations described herein. Considering embodiments in which hardware modules are temporarily configured (e.g., programmed), each of the hardware modules need not be configured or instantiated at any one instance in time. For example, where the hardware modules comprise a general-purpose processor configured using software, the general-purpose processor may be configured as respective different hardware modules at different times. Software may accordingly configure a processor, for example, to constitute a particular hardware module at one instance of time and to constitute a different hardware module at a different instance of time.

Hardware modules can provide information to, and receive information from, other hardware modules. Accordingly, the described hardware modules may be regarded as being communicatively coupled. Where multiple of such hardware modules exist contemporaneously, communications may be achieved through signal transmission (e.g., over appropriate circuits and buses) that connect the hardware modules. In embodiments in which multiple hardware modules are configured or instantiated at different times, communications between such hardware modules may be achieved, for example, through the storage and retrieval of information in memory structures to which the multiple hardware modules have access. For example, one hardware module may perform an operation and store the output of that operation in a memory device to which it is communicatively coupled. A further hardware module may then, at a later time, access the memory device to retrieve and process the stored output. Hardware modules may also initiate communications with input or output devices, and can operate on a resource (e.g., a collection of information).

The various operations of example methods described herein may be performed, at least partially, by one or more processors that are temporarily configured (e.g., by software) or permanently configured to perform the relevant operations. Whether temporarily or permanently configured, such processors may constitute processor-implemented modules that operate to perform one or more operations or functions. The modules referred to herein may, in some example embodiments, comprise processor-implemented modules.

Similarly, the methods or routines described herein may be at least partially processor-implemented. For example, at least some of the operations of a method may be performed by one or more processors or processor-implemented hardware modules. The performance of certain operations may be distributed among the one or more processors, not only residing within a single machine, but deployed across a number of machines. In some example embodiments, the processor or processors may be located in a single location (e.g., within a home environment, an office environment or as a server farm), while in other embodiments the processors may be distributed across a number of locations.

The performance of certain operations may be distributed among the one or more processors, not only residing within a single machine, but deployed across a number of machines. In some example embodiments, the one or more processors or processor-implemented modules may be located in a single geographic location (e.g., within a home environment, an office environment, or a server farm). In other example embodiments, the one or more processors or processor-implemented modules may be distributed across a number of geographic locations.

Unless specifically stated otherwise, discussions herein using words such as "processing," "computing, " "calculating, " "predicting," "proposing," determining, " "presenting," "displaying," or the like may refer to actions or processes of a machine (e.g., a computer) that manipulates or transforms data represented as physical (e.g., electronic, magnetic, or optical) quantities within one or more memories (e.g., volatile memory, non-volatile memory, or a combination thereof), registers, or other machine components that receive, store, transmit, or display information.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Some embodiments may be described using the expression "coupled" and "connected" along with their derivatives. For example, some embodiments may be described using the term "coupled" to indicate that two or more elements are in direct physical or electrical contact. The term "coupled," however, may also mean that two or more elements are not in direct contact with each other, but yet still cooperate or interact with each other. The embodiments are not limited in this context.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description, and the claims that follow, should be read to include one or at least one and the singular also includes the plural unless it is obvious that it is meant otherwise.

Although the preceding text sets forth a detailed description of numerous different embodiments, it should be understood that the legal scope of the invention is defined by the words of the claims set forth at the end of this patent. The detailed description is to be construed as example only and does not describe every possible embodiment, as describing every possible embodiment would be impractical, if not impossible. One could implement numerous alternate embodiments, using either current technology or technology developed after the filing date of this patent, which would still fall within the scope of the claims.

It should also be understood that, unless a term is expressly defined in this patent using the sentence "As used herein, the term ' _______________' is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term be limited, by implication or otherwise, to that single meaning.

## Claims

1. A method of generating a graphical user interface, the method comprising:
accessing, by one or more processors, a plurality of patient information, the patient information including time-sequenced data indicating events for a patient and hemoglobin measurements for the patient;
generating, by the one or more processors, one or more proposed treatment schedules, the treatment schedules indicating a proposed timeline at which events are proposed to occur;
accessing, by the one or more processors, a predictive model that predicts future hemoglobin levels for the patient along proposed timelines;
inputting, into the predictive model, the one or more proposed treatment schedules to determine a predicted trend for hemoglobin levels for the patient respectively associated with the one or more proposed treatment schedules;
displaying, on the graphical user interface, a graph indicating a visual representation of the patient information for the patient, the one or more proposed treatment schedules, and the predicted trends for the hemoglobin levels respectively associated with the proposed treatment schedules.

2. The method of claim 1, further comprising:
receiving, at the one or more processors, measured patient information associated with an event included in a treatment schedule selected from the one or more proposed treatment schedules.

3. The method of any one of the preceding claims, wherein:
the predictive model is developed to model an expected response for a population; and
receiving the measured patient information causes the one or more processors to adapt the predictive model to the patient.

4. The method of any one of the preceding claims wherein:
the predictive model is a stochastic model associated with a set of parameters; and
adapting the predictive model includes applying Bayesian prediction and fitting techniques to adjust the set of parameters.

5. The method of any one of the preceding claims, wherein the Bayesian predication and fitting techniques include particle filtering techniques.

6. The method of any one of the preceding claims, wherein:
parameters within the set of parameters are associated with a variance; and
adapting the predictive model reduces the variance associated with the parameters.

7. The method of any one of the preceding claims, further comprising:
inputting, by the one or more processors, a treatment strategy other than the selected treatment strategy into the adapted predictive model utilizing patient information as of a historical point in time; and
comparing, by the one or more processors, the patient information measured after the historical point in time to the predicted trend for hemoglobin levels generated by the adapted predictive model to determine an effectiveness of the selected treatment schedule.

8. The method of any one of the preceding claims, further comprising:
receiving, via the graphical user interface, an adjustment to a particular proposed treatment schedule, wherein the adjustment is at least one of an inclusion of an event, a deletion of an event, a shift in when an event occurs, or a change in a type of event.

9. The method of any one of the preceding claims, further comprising:
responsive to receiving the adjustment:
inputting, by the one or more processors, the adjusted proposed treatment schedule into the predictive model; and
based on the output of the predictive model, dynamically updating, by the one or more processors, the predicted trend for the hemoglobin levels associated with the particular proposed treatment schedule.

10. The method of any one of the preceding claims, wherein:
the one or more processors include a first set of processors that are a component of a first computer system and a second set of processors that are a component of a second computer system;
the first computer system is specifically configured to execute and adapt the predictive model; and
the second computer system is configured to display the graphical user interface.

11. A system for generating a graphical user interface, the system comprising:
one or more processors;
a graphical user interface;
a non-transitory memory device operatively coupled to the one or more processors; and
one or more instructions stored on the memory, which when executed by the one or more processors, cause the system to:
access a plurality of patient information, the patient information including time-sequenced data indicating events for a patient and hemoglobin measurements for the patient;
generate one or more proposed treatment schedules, the treatment schedules indicating a proposed timeline at which events are proposed to occur;
access a predictive model that predicts future hemoglobin levels for the patient along proposed timelines;
input the one or more proposed treatment schedules to determine a predicted trend for hemoglobin levels for the patient respectively associated with the one or more proposed treatment schedules;
displaying, on the graphical user interface, a graph indicating a visual representation of the patient information for the patient, the one or more proposed treatment schedules, and the predicted trends for the hemoglobin levels respectively associated with the proposed treatment schedules.

12. The system of claim 11, wherein the instructions, when executed by the one or more processors, further cause the system to:
receive measured patient information associated with an event included in a treatment schedule selected from the one or more proposed treatment schedules.

13. The system of any one of claims 11-12, wherein:
the predictive model is developed to model an expected response for a population; and
the instructions, when executed by the one or more processors, further cause the system to adapt the predictive model to the patient based on the measured patient information.

14. The system of any one of claims 11-13, wherein:
the predictive model is associated with a set of parameters; and
to adapt the predictive model, the instructions, when executed by the one or more processors, further cause the system apply particle filtering to adjust the set of parameters.

15. The system of any one of claims 11-14, wherein the instructions, when executed by the one or more processors, further cause the system to:
input a treatment strategy other than the selected treatment strategy into the adapted predictive model utilizing patient information as of a historical point in time; and
compare the patient information measured after the historical point in time to the predicted trend for hemoglobin levels generated by the adapted predictive model to determine an effectiveness of the selected treatment schedule.

## Patentansprüche

1. Verfahren zum Erzeugen einer graphischen Benutzeroberfläche, wobei das Verfahren umfasst:
Zugreifen durch einen oder mehrere Prozessoren auf eine Mehrzahl von Patienteninformationen, wobei die Patienteninformationen zeitlich sequenzierte Daten beinhalten, die Ereignisse für einen Patienten und Hämoglobinmessungen für den Patienten anzeigen;
Erzeugen durch den einen oder die mehreren Prozessoren von einem oder mehreren vorgeschlagenen Behandlungsplänen, wobei die Behandlungspläne eine vorgeschlagene Zeitlinie angeben, zu der Ereignisse auftreten sollen;
Zugreifen durch den einen oder die mehreren Prozessoren auf ein Vorhersagemodell, das zukünftige Hämoglobinspiegel für den Patienten entlang vorgeschlagener Zeitlinien vorhersagt;
Eingeben des einen oder der mehreren vorgeschlagenen Behandlungspläne in das Vorhersagemodell, um einen vorhergesagten Trend für Hämoglobinspiegel für den Patienten zu bestimmen, der jeweils dem einen oder den mehreren vorgeschlagenen Behandlungsplänen zugeordnet ist;
Anzeigen eines Diagramms auf der grafischen Benutzeroberfläche, das eine visuelle Darstellung der Patienteninformationen für den Patienten, des einen oder der mehreren vorgeschlagenen Behandlungspläne und der vorhergesagten Trends für die Hämoglobinspiegel anzeigt, die jeweils den vorgeschlagenen Behandlungsplänen zugeordnet sind.

2. Verfahren nach Anspruch 1, das ferner umfasst:
Empfangen in dem einen oder den mehreren Prozessoren von gemessenen Patienteninformationen, die einem Ereignis zugeordnet sind, das in einem Behandlungsplan beinhaltet ist, der aus dem einen oder den mehreren vorgeschlagenen Behandlungsplänen ausgewählt ist.

3. Verfahren nach einem der vorhergehenden Ansprüche, wobei:
das Vorhersagemodell entwickelt wird, um eine erwartete Reaktion für eine Population zu modellieren; und
das Empfangen der gemessenen Patienteninformation den einen oder die mehreren Prozessoren veranlasst, das Vorhersagemodell an den Patienten anzupassen.

4. Verfahren nach einem der vorhergehenden Ansprüche, wobei:
das Vorhersagemodell ein stochastisches Modell ist, das einem Satz von Parametern zugeordnet ist; und
das Anpassen des Vorhersagemodells die Anwendung von Bayes'schen Vorhersage- und Anpassungstechniken zum Anpassen des Satzes von Parametern beinhaltet.

5. Verfahren nach einem der vorhergehenden Ansprüche, wobei die Bayes'schen Vorhersage- und Anpassungstechniken Partikelfiltertechniken beinhalten.

6. Verfahren nach einem der vorhergehenden Ansprüche, wobei:
Parameter innerhalb des Satzes von Parametern einer Varianz zugeordnet sind; und
das Anpassen des Vorhersagemodells die den Parametern zugeordnete Varianz reduziert.

7. Verfahren nach einem der vorhergehenden Ansprüche, ferner umfassend:
Eingeben durch den einen oder die mehreren Prozessoren einer anderen als der ausgewählten Behandlungsstrategie in das angepasste Vorhersagemodell unter Verwendung von Patienteninformationen zu einem historischen Zeitpunkt; und
Vergleichen durch den einen oder die mehreren Prozessoren der nach dem historischen Zeitpunkt gemessenen Patienteninformationen mit dem durch das angepasste Vorhersagemodell erzeugten vorhergesagten Trend für Hämoglobinspiegel, um eine Wirksamkeit des ausgewählten Behandlungsplans zu bestimmen.

8. Verfahren nach einem der vorhergehenden Ansprüche, ferner umfassend:
Empfangen einer Anpassung eines bestimmten vorgeschlagenen Behandlungsplans über die grafische Benutzerschnittstelle, wobei die Anpassung wenigstens eines von einem Einschluss eines Ereignisses, einer Löschung eines Ereignisses, einer Verschiebung des Zeitpunkts, zu dem ein Ereignis auftritt, oder einer Änderung einer Art von Ereignis ist.

9. Verfahren nach einem der vorhergehenden Ansprüche, ferner umfassend:
als Reaktion auf den Empfang der Anpassung:
Eingeben durch den einen oder die mehreren Prozessoren des angepassten vorgeschlagenen Behandlungsplans in das Vorhersagemodell; und
auf Grundlage der Ausgabe des Vorhersagemodells, dynamisches Aktualisieren durch den einen oder die mehreren Prozessoren des vorhergesagten Trends für die Hämoglobinspiegel, die dem bestimmten vorgeschlagenen Behandlungsplan zugeordnet sind.

10. Verfahren nach einem der vorhergehenden Ansprüche, wobei:
der eine oder die mehreren Prozessoren einen ersten Satz von Prozessoren, die eine Komponente eines ersten Computersystems sind, und einen zweiten Satz von Prozessoren beinhalten, die eine Komponente eines zweiten Computersystems sind;
das erste Computersystem speziell dazu konfiguriert ist, das Vorhersagemodell auszuführen und anzupassen; und
das zweite Computersystem dazu konfiguriert ist, die grafische Benutzeroberfläche anzuzeigen.

11. System zum Erzeugen einer grafischen Benutzerschnittstelle, wobei das System umfasst:
einen oder mehrere Prozessoren;
eine grafische Benutzeroberfläche;
eine nichtflüchtige Speichervorrichtung, die betriebsmäßig mit dem einen oder den mehreren Prozessoren gekoppelt ist; und
eine oder mehrere Anweisungen, die in dem Speicher gespeichert sind, die, wenn sie von dem einen oder den mehreren Prozessoren ausgeführt werden, das System veranlassen, zum:
Zugreifen auf eine Mehrzahl von Patienteninformationen, wobei die Patienteninformationen zeitlich sequenzierte Daten beinhalten, die Ereignisse für einen Patienten und Hämoglobinmessungen für den Patienten anzeigen;
Erzeugen von einem oder mehreren vorgeschlagenen Behandlungsplänen, wobei die Behandlungspläne eine vorgeschlagene Zeitlinie angeben, zu der Ereignisse auftreten sollen;
Zugreifen auf ein Vorhersagemodell, das zukünftige Hämoglobinspiegel für den Patienten entlang vorgeschlagener Zeitlinien vorhersagt;
Eingeben des einen oder der mehreren vorgeschlagenen Behandlungspläne in das Vorhersagemodell, um einen vorhergesagten Trend für Hämoglobinspiegel für den Patienten zu bestimmen, der jeweils dem einen oder den mehreren vorgeschlagenen Behandlungsplänen zugeordnet ist;
Anzeigen eines Diagramms auf der grafischen Benutzeroberfläche, das eine visuelle Darstellung der Patienteninformationen für den Patienten, des einen oder der mehreren vorgeschlagenen Behandlungspläne und der vorhergesagten Trends für die Hämoglobinspiegel anzeigt, die jeweils den vorgeschlagenen Behandlungsplänen zugeordnet sind.

12. System nach Anspruch 11, wobei die Anweisungen, wenn sie von dem einen oder den mehreren Prozessoren ausgeführt werden, das System ferner dazu veranlassen, zum:
Empfangen gemessener Patienteninformationen, die einem Ereignis zugeordnet sind, das in einem Behandlungsplan beinhaltet ist, der aus dem einen oder den mehreren vorgeschlagenen Behandlungsplänen ausgewählt ist.

13. System nach einem der Ansprüche 11-12, wobei:
das Vorhersagemodell entwickelt wird, um eine erwartete Reaktion für eine Population zu modellieren; und
die Anweisungen, wenn sie von dem einen oder den mehreren Prozessoren ausgeführt werden, ferner das System veranlassen, das Vorhersagemodell auf Grundlage der gemessenen Patienteninformationen an den Patienten anzupassen.

14. System nach einem der Ansprüche 11-13, wobei:
das Vorhersagemodell einem Satz von Parametern zugeordnet ist; und
um das Vorhersagemodell anzupassen, die Anweisungen, wenn sie von dem einen oder den mehreren Prozessoren ausgeführt werden, ferner das System veranlassen, eine Partikelfilterung anzuwenden, um den Satz von Parametern anzupassen.

15. System nach einem der Ansprüche 11-14, wobei die Anweisungen, wenn sie von dem einen oder den mehreren Prozessoren ausgeführt werden, das System ferner veranlassen, zum:
Eingeben einer anderen Behandlungsstrategie als der ausgewählten Behandlungsstrategie in das angepasste Vorhersagemodell unter Verwendung von Patienteninformationen ab einem historischen Zeitpunkt; und
Vergleichen der nach dem historischen Zeitpunkt gemessenen Patienteninformationen mit dem Patienteninformation, die nach dem historischen Zeitpunkt gemessen wurde, mit dem vorhergesagten Trend für Hämoglobinwerte, der durch das angepasste Vorhersagemodell erzeugt wurde, um eine Wirksamkeit des ausgewählten Behandlungsplans zu bestimmen.

## Revendications

1. Procédé de génération d'une interface utilisateur graphique, le procédé comprenant :
l'accès, par un ou plusieurs processeurs, à une pluralité d'informations de patient, les informations de patient comprenant des données séquencées dans le temps indiquant des événements pour un patient et des mesures d'hémoglobine pour le patient ;
la génération, par l'un ou plusieurs processeurs, d'un ou plusieurs programmes de traitement proposés, les programmes de traitement indiquant un calendrier proposé auquel des événements sont proposés pour se produire ;
l'accès, par l'un ou plusieurs processeurs, à un modèle prédictif qui prédit les niveaux futurs d'hémoglobine pour le patient selon des délais proposés ;
la saisie, dans le modèle prédictif, de l'un ou plusieurs programmes de traitement proposés pour déterminer une tendance prédite des niveaux d'hémoglobine pour le patient respectivement associée à l'un ou plusieurs programmes de traitement proposés ;
l'affichage, sur l'interface utilisateur graphique, d'un graphique indiquant une représentation visuelle des informations de patient pour le patient, l'un ou plusieurs programmes de traitement proposés, et les tendances prédites pour les taux d'hémoglobine respectivement associés aux programmes de traitement proposés.

2. Procédé selon la revendication 1, comprenant en outre :
la réception, au niveau de l'un ou plusieurs processeurs, d'informations de patient mesurées associées à un événement inclus dans un programme de traitement sélectionné parmi l'un ou plusieurs programmes de traitement proposés.

3. Procédé selon l'une quelconque des revendications précédentes, dans lequel :
le modèle prédictif est développé pour modéliser une réponse attendue pour une population ; et
la réception des informations de patient mesurées amène l'un ou plusieurs processeurs à adapter le modèle prédictif au patient.

4. Procédé selon l'une quelconque des revendications précédentes dans lequel :
le modèle prédictif est un modèle stochastique associé à un ensemble de paramètres ; et
l'adaptation du modèle prédictif comprend l'application de techniques de prédiction et d'ajustement bayésiennes pour ajuster l'ensemble de paramètres.

5. Procédé selon l'une quelconque des revendications précédentes, dans lequel les techniques de prédiction et d'ajustement bayésiennes comprennent des techniques de filtrage de particules.

6. Procédé selon l'une quelconque des revendications précédentes, dans lequel :
des paramètres dans l'ensemble de paramètres sont associés à une variance ; et
l'adaptation du modèle prédictif réduit la variance associée aux paramètres.

7. Procédé selon l'une quelconque des revendications précédentes, comprenant en outre :
l'entrée, par l'un ou plusieurs processeurs, d'une stratégie de traitement autre que la stratégie de traitement sélectionnée dans le modèle prédictif adapté utilisant les informations de patient à un point historique dans le temps ; et
la comparaison, par l'un ou plusieurs processeurs, des informations de patient mesurées après le point historique dans le temps à la tendance prédite des niveaux d'hémoglobine générée par le modèle prédictif adapté pour déterminer une efficacité du programme de traitement sélectionné.

8. Procédé selon l'une quelconque des revendications précédentes, comprenant en outre :
la réception, via l'interface utilisateur graphique, d'un ajustement à un programme de traitement proposé particulier, dans lequel l'ajustement est au moins l'un parmi une inclusion d'un événement, une suppression d'un événement, un changement dans le moment où un événement se produit ou un changement dans un type d'événement.

9. Procédé selon l'une quelconque des revendications précédentes, comprenant en outre :
en réponse à la réception de l'ajustement :
l'entrée, par l'un ou plusieurs processeurs, du programme de traitement proposé ajusté dans le modèle prédictif ; et
sur la base de la sortie du modèle prédictif, la mise à jour dynamique, par l'un ou plusieurs processeurs, de la tendance prédite pour les taux d'hémoglobine associés au programme de traitement proposé particulier.

10. Procédé selon l'une quelconque des revendications précédentes, dans lequel :
l'un ou plusieurs processeurs comprennent un premier ensemble de processeurs qui sont un composant d'un premier système informatique et un deuxième ensemble de processeurs qui sont un composant d'un deuxième système informatique ;
le premier système informatique est spécifiquement configuré pour exécuter et adapter le modèle prédictif ; et
le deuxième système informatique est configuré pour afficher l'interface utilisateur graphique.

11. Système de génération d'une interface utilisateur graphique, le système comprenant :
un ou plusieurs processeurs ;
une interface utilisateur graphique ;
un dispositif de mémoire non transitoire couplé fonctionnellement à l'un ou plusieurs processeurs ; et
une ou plusieurs instructions stockées dans la mémoire, qui, lorsqu'elles sont exécutées par l'un ou plusieurs processeurs, amènent le système à :
accéder à une pluralité d'informations de patient, les informations de patient comprenant des données séquencées dans le temps indiquant des événements pour un patient et des mesures d'hémoglobine pour le patient ;
générer un ou plusieurs programmes de traitement proposés, les programmes de traitement indiquant un calendrier proposé auquel des événements sont proposés pour se produire ;
accéder à un modèle prédictif qui prédit les niveaux futurs d'hémoglobine pour le patient selon les délais proposés ;
saisir l'un ou plusieurs programmes de traitement proposés pour déterminer une tendance prédite des niveaux d'hémoglobine pour le patient respectivement associée à l'un ou plusieurs programmes de traitement proposés ;
afficher, sur l'interface utilisateur graphique, un graphique indiquant une représentation visuelle des informations de patient pour le patient, l'un ou plusieurs programmes de traitement proposés, et les tendances prédites pour les taux d'hémoglobine respectivement associés aux programmes de traitement proposés.

12. Système selon la revendication 11, dans lequel les instructions, lorsqu'elles sont exécutées par l'un ou plusieurs processeurs, amènent en outre le système à :
recevoir des informations de patient mesurées associées à un événement inclus dans un programme de traitement sélectionné parmi l'un ou plusieurs programmes de traitement proposés.

13. Système selon l'une quelconque des revendications 11 à 12, dans lequel :
le modèle prédictif est développé pour modéliser une réponse attendue pour une population ; et
les instructions, lorsqu'elles sont exécutées par l'un ou plusieurs processeurs, amènent en outre le système à adapter le modèle prédictif au patient sur la base des informations de patient mesurées.

14. Système selon l'une quelconque des revendications 11 à 13, dans lequel :
le modèle prédictif est associé à un ensemble de paramètres ; et
pour adapter le modèle prédictif, les instructions, lorsqu'elles sont exécutées par l'un ou plusieurs processeurs, amènent en outre le système à appliquer un filtrage de particules pour ajuster l'ensemble de paramètres.

15. Système selon l'une quelconque des revendications 11 à 14, dans lequel les instructions, lorsqu'elles sont exécutées par l'un ou plusieurs processeurs, amènent en outre le système à :
saisir une stratégie de traitement autre que la stratégie de traitement sélectionnée dans le modèle prédictif adapté utilisant les informations du patient à partir d'un point historique dans le temps ; et
comparer les informations de patient mesurées après le point historique dans le temps à la tendance prédite des niveaux d'hémoglobine générés par le modèle prédictif adapté pour déterminer une efficacité du programme de traitement sélectionné.
